# EUROPEAN PATENT SPECIFICATION

(11) **EP 1 289 557 B1**
(45) Date of publication and mention of the grant of the patent: **12.07.2006**
(21) Application number: 01941917.5
(22) Date of filing: 04.06.2001
(51) Int. Cl.: A61K 45/00

(54) **CANCER TREATMENT COMPOSITION CONTAINING AN ANTI-NEOPLASTIC AGENT AND A PDE4 INHIBITOR**
KREBSBEHANDLUNG ZUSAMMENSETZUNG, WELCHE EIN ANTINEOPLASTISCHES MITTEL UND PDE4 INHIBITOR ENTHÄLT
COMPOSITION POUR LE TRAITEMENT DU CANCER COMPRENANT UN AGENT ANTI-NEOPLASTIQUE ET UN INHIBITEUR DE PDE4

(30) Priority: 06.06.2000 US 209846 P
(43) Date of publication of application: 12.03.2003
(73) Proprietor: GLAXO GROUP LIMITED, Greenford, Middlesex UB6 0NN (GB)
(72) Inventor: DAVIS, Stephen, Thomas, Durham, NC 27703 (US); DEV, Inderjit, Kumar, Research Triangle Park, NC 27709 (US); DORNSIFE, Ronna, Ellen, Research Triangle Park, NC 27709 (US); GRAY, John, Gordon, Research Triangle Park, NC 27709 (US)
(74) Representative: Valentine, Jill Barbara
(86) International application number: PCT/US2001/018108
(87) International publication number: WO 2001/093909

(56) References cited:
- EP-A- 1 188 438
- WO-A-00/16621
- WO-A-01/78651
- WO-A-98/41232
- WO-A-99/34804
- WO-A-99/49859
- DORNSIFE RONNA E ET AL: "Phosphodiesterase 4 (PDE4) inhibitors provide selective chemoprotection of human neutrophil progenitors from phase-specific anti-cancer agents with no effect on anti-neoplastic potency." BLOOD, vol. 96, no. 11 Part 1, 16 November 2000 (2000-11-16), page 20a XP001074197 42nd Annual Meeting of the American Society of Hematology;San Francisco, California, USA; December 01-05, 2000 ISSN: 0006-4971
- DORNSIFE RONNA E ET AL: "Selective chemoprotection of human neutrophil progenitors and mink lung epithelial cells by phosphodiesterase 4 (PDE4) inhibitors in vitro from the toxicities of phase-specific anti-cancer agents with no effect on anti-neoplastic potency." PROCEEDINGS OF THE AMERICAN ASSOCIATION FOR CANCER RESEARCH ANNUAL, vol. 42, March 2001 (2001-03), page 189 XP008007511 92nd Annual Meeting of the American Association for Cancer Research;New Orleans, LA, USA; March 24-28, 2001, March, 2001 ISSN: 0197-016X
- PIAZZA G A ET AL: "Exisulind, a novel proapoptotic drug, inhibits rat urinary bladder tumorigenesis." CANCER RESEARCH. UNITED STATES 15 MAY 2001, vol. 61, no. 10, 15 May 2001 (2001-05-15), pages 3961-3968, XP001104534 ISSN: 0008-5472

## Description

### BACKGROUND OF THE INVENTION

The present invention relates to a method of treating cancer in a mammal and to pharmaceutical combinations useful in such treatment In particular, the method relates to a cancer treatment method that includes administering anti-neoplastic agents which adversely effect normal progenitor cells and phosphodiesterase 4 (PDE4) inhibitors to the mammal. Such PDE4 inhibitors attenuate unwanted side effects on normal progenitor cells of the mammal resulting from administration of said anti-neoplastic agent.

Effective chemotherapy for cancer treatment, which has acceptable toxicity to normal cells, is a continuing goal in the oncology field. Numerous agents are used in the treatment of cancer, including agents that adversely affect normal progenitor cells upon use. One such class of anti-neoplastic agents that have an adverse effect on normal progenitor cells is known as phase specific anti-neoplastic agents. Phase specific anti-neoplastic agents have activity against cells that are in the process of cell division. Typically, such agents have effect on the cell cycle at G₁ - the period between mitosis and DNA synthesis; S - the period of DNA synthesis; G₂ - the premitotic interval; and/or M - the period of mitosis. These agents are not effective in Go, the quiescent or resting cell phase. Therefore, phase specific anti-neoplastic agents are active against cells in the process of cell division. Phase specific anti-neoplastic agents are most effective against cancers that have a large growth fraction, that is, tumors that have a high percentage of dividing cells. Problematically, however, such agents also have an adverse effect on rapidly proliferating, normal tissues such as bone marrow, hair follicles and intestinal epithelium. (See Goodman Et Gilman's, The Pharmacologic Basis Of Therapeutics 9^{th} Ed., pages 1230-1232.)

As indicated above, the bone marrow and epithelial mucosa (gastrointestinal and oral) have a rapid cell cycle and are particularly sensitive to phase specific anti-neoplastic agents. Consequently, myelosuppression and mucositis are the two most common dose-limiting side effects of anti-neoplastic treatment Myelosuppression is suppression of normal bone marrow activity (hematopoiesis) that ultimately causes a reduction in white blood cells (neutropenia), platelets (thrombocytopenia), and/or red blood cells (anemia) in the peripheral blood. All of these adverse effects can lead to complications to the patient's health. For instance, neutropenia can lead to susceptibility to recurrent and life threatening, opportunistic, bacterial infections. Mucositis is inflammation resulting from damage to the epithelial mucosae. Mucositis induced by anti-neoplastic therapies includes damage to the epithelial barrier and potential entry of microbial flora into the blood. Neutropenia and mucositis, if severe enough, limit patients' ability to tolerate subsequent courses of anti-neoplastic drugs (Kim SK, Demitri GD, Hematology/Oncology Clinics of North America, 10 (2) 377-393, (1996)).

While effective treatment for mucositis is still a major unmet medical need and continues to be a threat to patients treated with anti-neoplastic agents, several techniques have been utilized to reduce and/or control myelosuppression. Patients with neutropenia are commonly treated with rescue agents such as granulocyte-colony stimulating factor (G-CSF) to reduce the duration and severity of neutropenia. Although this approach has been proven to reduce peripheral neutropenia, no survival benefit to patients from either reduced infection or dose-intensification of chemotherapy has been demonstrated (Higa, GD; Pharmacotherapy, 18 (6) 1249-1254, (1998); Johnston, E; Crawford, J; Seminars in Oncology, 25 (5): 552-561, (1998); Petros, WP; Crawford, J; Current Opinion in Hematology, 4 (3): 213-216). Moreover, recent evidence suggests that stimulation by G-CSF forces surviving stem cells into proliferation and repeated rounds of chemotherapy further damages and depletes stem cell pools. (van Os, R et al., Blood, 92 (6), 1950-1956, (1997); Higa, GD; Pharmacotherapy, 18 (6) 1249-1254, (1998); Johnston, E; Crawford, J; Seminars in Oncology, 25 (5) 552-561, (1998)).

Instead of stimulating proliferation of damaged and/or surviving stem cells, a strategy of total or partial preservation of normal cells by chemoprotection would be a superior approach. Many investigators have suggested that stem cells can be protected from radiation and chemotherapy damage by keeping the stem cells in a quiescent state during drug exposure. Numerous studies have demonstrated that elevated concentration of intracellular cAMP in certain cell types result in suppression of cell proliferation and activation. (Vairo, G et al., J Biol Chem, 265 (6): 2692-2700, (1990); Berridge, MV et al., Experimental Hematology, 21: 269-276 (1993); Morley, A et al., Proc Soc Exp Biol Med, 138: 57-59, (1997); Ralph, RK, FEBS lett, 161: 1-8). Increased intracellular cAMP also increases the survival and half-life of mature peripheral blood neutrophils apparently by decreasing natural apoptosis (Walker, BA et al., J Immunol, 158: 2926-2931; Rossi, AG, Biochem Biophys Research Commun, 217 (3), 892-899 (1995); Niwa, M et al. Euro J Pharmacol 371, 59-67, (1999)).

In WO9949859 the non-steroidal anti-inflammatory agent sulindac, which is also a PDE4 inhibitor, in combination with the anticancer agent difluoromethylomithine was proposed for the prevention/treatment of cancers characterised by the expression of an activated Ki-ras, and in WO0016621, a PDE4 specific inhibitor in combination with chemotherapeutic agents was disclosed as a potential method for treating chronic lymphocytic leukaemia. In WO0178651, an antineoplastic topoisomerase I inhibitor in combination with a PDE inhibitor was disclosed as a potential treatment for neoplasia, whereas EP-A-1 188438 related to the proposed use of PDE4 inhibitors for manufacturing a medicament for preventing Or treating a disease associated with an excess of IL-12 production.

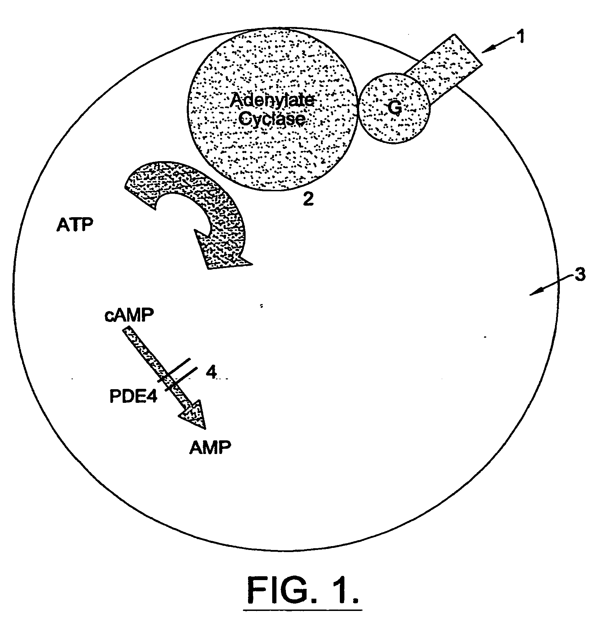

The present inventors hypothesized that elevated levels of intracellular cAMP might protect normal progenitor and stem cells in various tissues from the cytotoxicities produced by anti-neoplastic agents through induction of cell cycle arrest and/or by inhibition of apoptosis. Intracellular concentrations of cAMP can be elevated by several mechanisms (see Figure 1), including activating surface receptors which are G-protein coupled to the cAMP generating enzyme adenyl cyclase (e.g. protaglandin E-2 [PGE2]) 1; increasing cAMP levels by diredly stimulating adenylate cyclase (e.g. forskolin) 2; by treating with a stable analog of cAMP (e.g., dibutyl CAMP, 8-Cl CAMP, or 8-bromo cAMP) 3, or by inhibiting cyclic nucleotide phosphodiesterases (PDEs), which decreases the rate at which cAMP is catabolized to AMP 4.

Phosphodiesterases (PDEs) comprise a large, divergent family of enzymes that catalyze the catabolism of cAMP and cGMP to AMP and GMP, respectively. Eleven members of this family have been identified to date based on substrate specificity, kinetic properties, sensitivity to specific inhibitors, tissue distribution, and primary sequences (Manganiello, VC et al., Arch Biochem Biophys, 322 (1): 1-13, (1995);
Fawcett, L et al., Proc Natl Acad Sci USA, 97(7): 3702-3707 (2000)). Recognition of these differences has greatly stimulated interest in PDEs as drug targets. In particular, the cAMP specific PDE, termed PDE4, which is found in nearly all immune and inflammatory cells is an attractive target for novel anti-asthmatic and anti-inflammatory therapies.

The present inventors have now discovered a new method of treating cancer using a novel pharmaceutical combination, which can selectively treat cancers, while attenuating the associated adverse, side effects including myelosuppression and mucositis. The present invention recognizes the use of PDE4 inhibitors as selective agents that could provide protection from myelosuppression and mucositis induced by anti-neoplastic agents without affecting the anti-neoplastic efficacy of these drugs. Elevation of cAMP by inhibiting PDE4 isoenzyme offers a definite advantage over inhibiting multiple PDE isoenzymes because it can potentially lead to an increase of intracellular cAMP in normal cells, but not in tumor cells. PDE4 is the exclusive cAMP PDE isoenzyme found in neutrophils and is the predominant cAMP PDE isoenzyme in epithelial cells. Selective inhibition of PDE4 in neutrophils or epithelial cells would raise the intracellular level of cAMP in those cells significantly. In contrast, tumor cells have other cAMP PDE isoenzymes in addition to PDE4. These additional isoenzymes in tumor cells would allow cAMP to be catabolized to AMP and thus prevent a buildup of intracellular cAMP even if treated with a selective PDE4 inhibitor.

### SUMMARY OF THE INVENTION

In a first aspect of the present invention, there is provided a cancer treatment combination, comprising: therapeutically effective amounts of
(i) an anti-neoplastic agent that adversely affects normal progenitor cells upon use selected from paclitaxel, docetaxel, topotecan, irinotecan, 7-(4-methylpiperazino-methylene)-10,11-ethylenedioxy-20-camptothecin, vinblastine, vincristine, vinorelbine, etoposide, teniposide, fluorouracil, methotrexate, cytarabine, mercaptopurine, thioguanine, floxuridine, 5-fluorodeoxyuridine monophosphate, 5-azacytidine, gemcitabine, pentostatin, erythrohydroxynonyladenine, fludarabine phosphate and cladribine;
(ii) a PDE4 inhibitor selected from cis-4-cyano-4-[3-(cyclopentyloxy)-4-methoxyphenyl)cyclohexane-1-carboxylic acid or salt thereof [Ariflo™], N-(3,5-dichloropyrid-4-yl)-3-cyclopentyloxy-4-methoxybenzamide [piclamilast], 1-[3-cyclopentyloxy)-4-methoxyphenyl) ethanone (E)-O-{aminocarbonyl)oxime [filaminast], (+/-)-4-[2-(3-Cyclopentyloxy-4-methoxyphenyl)-2-phenylethyl]pyridine [CDP-840], nitroquazone, rolipram, denbufylline, 8-aza-1-(3-nitrophenyl)-3-(4-pyridylmethyl-2,4-quinazolinedione [RS-25344], 1-(3-carbomethoxyphenyl) -3-benzylpyrido [2,3d] pyrimidine-2,4(1H,3H)dione [CP-77059] and methyl (3S,4S)-3-acetyl-[3-(cyclopentyloxy)-4-methyloxy)phenyl]-3-methyl-pyrrolidinecarboxylate [GI193600X]; and
(iii) an agent effective in raising intracellular concentrations of cAMP or analogs thereof selected from forskolin, dibutyryl CAMP, 8-bromo CAMP, terbutaline, albuterol, pirbuterol, bitolterol, metaproterenol, formoterol and salmeterol or salt thereof.

### BRIEF DESCRIPTION OF THE DRAWINGS

Figure 1 depicts various mechanisms for increasing intracellular concentrations of cAMP in cells.
Figure 2 depicts a flow cytometric evaluation of expanded CD34° stem cells treated with (a) DMSO; (b) rolipram plus forskolin followed by treatment with various doses of paclitaxel; and (c) a plot of percent live cells vs. paclitaxel dose with preteatment with DMSO or rolipram plus forskolin.
Figure 3 (a), (b), and (c) depict protection of specific-stage neutrophil progenitors (CFU-GMs) as measured by colony forming assay wherein cells are treated with rolipram plus forskolin followed by treatment with either (a) 5-fluorouracil, (b) etoposide, or (c) paclitaxel.
Figure 4 (a), (b), (c), (d), (e), (f), and (g) depict protection of multiple-stage neutrophil progenitors as measured by a CD15 neutrophil antigen-cell-based ELISA (CELISA) method wherein cells are treated with rolipram plus forskolin followed by treatment with either (a) paclitaxel, (b) 5-fluorouracil, (c) etoposide, (d) vinblastine, (e) vinorelbine, (f) methotrexate, or (g) topotecan.

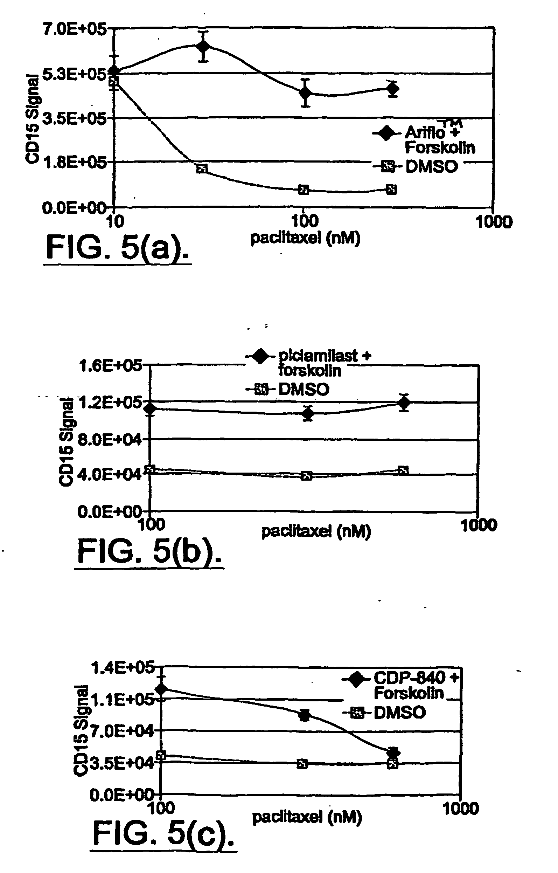

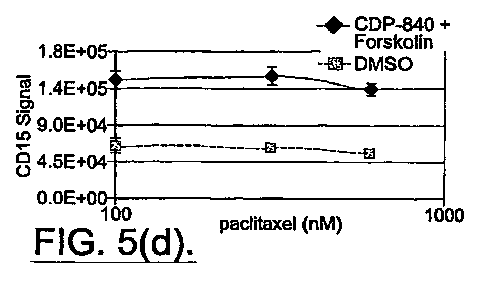

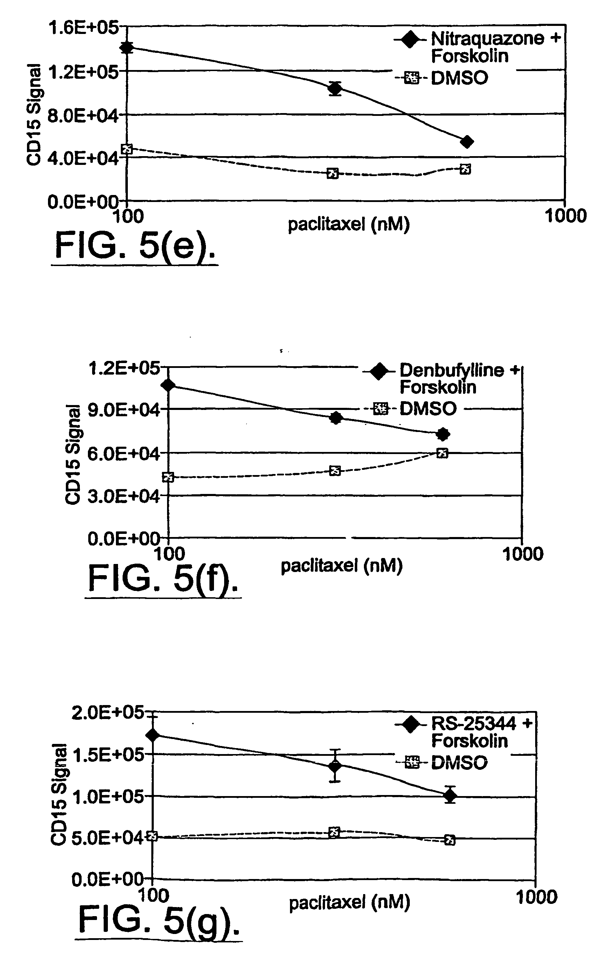

Figure 5 (a), (b), (c), (d), (e), (f), (g), (h), and (i) depict protection of multiple-stage neutrophil progenitors as measured by a CD15 neutrophil antigen-cell-based ELISA (CELISA) method wherein cells are treated with (a) Ariflo™, (b) Piclamilast, (c) CDP-840 [GR259653X], (d) CDP-840 [GR259654X], (e) Nitraquazone, (f) Denbufylline, (g) RS-25344 [8-aza-1-(3-nitrophenyl)-3(4-pyridylmethyl)2,4-quinazolinedione], (h) CP-77059 1-(3-carbomethoxyphenyl)-3-benzylpyrido [2,3d] pyrimidine-2,4(1H,3H)dione or (i) GI193600X [methyl (3S,4S)-3-acetyl-4-[3-(cyclopentyloxy)-4-(methyloxy)phenyl)-3-methyl-1-pyrrolidinecarboxylate], each plus forskolin followed by treatment with paclitaxel.
Figure 6 depicts protection of multiple-stage neutrophil progenitors as measured by a CD15 neutrophil antigen-cell-based ELISA (CELISA) method wherein cells are treated with Ariflo™ plus forskolin followed by treatment with topotecan.
Figure 7 (a), (b), and (c) depict non-protection of multiple-stage neutrophil progenitors as measured by a CD15 neutrophil-cell-based ELISA (CELISA) method wherein cells are treated with rolipram plus forskolin followed by treatment with either (a) cisplatin, (b) doxorubicin, or (c) carboplatin.
Figure 8 (a), (b), (c), (d), (e), (f), (g), and (h) depict no-effect on the anti-neoplastic effect of paclitaxel against either the human RKO colon tumor line ((a) and (c)) or the human HT-29 colon tumor line ((b) and (d)), or the human MDA468 breast tumor line ((e) and (g)) or the human A549 lung tumor line ((f) and (h)) wherein relative growth of tumor cells is measured with the protein stain sulforhodamine (SRB) after cells are treated with rolipram plus forskolin or Ariflo™ plus forskolin followed by treatment with 100 nM paclitaxel.
Figure 9 (a) and (b) depict PDE4 inhibitor compounds, (a) Ariflo™ and (b) piclamilast, useful in the present invention.
Figure 10 (a) and (b) depict PDE4 inhibitor compounds, (a) filaminast and (b) CDP-840, useful in the present invention.
Figure 11 (a), (b), and (c) depict PDE4 inhibitor compounds, (a) nitraquazone, (b) denbufylline, and (c) rolipram useful in the present invention.
Figure 12 depicts PDE4 inhibitor compound Gl193600X, useful in the present invention.

### DETAILED DESCRIPTION OF THE INVENTION

As used herein, the term "alkyl" refers to a straight or branched chain hydrocarbon having from one to twelve carbon atoms, optionally substituted with substituents selected from the group which includes C₁-C₆ alkyl, C₁-C₆ alkoxy, C₁-C₆ alkylsulfanyl, C₁-C₆ alkylsulfenyl, C₁-C₆ alkylsulfonyl, oxo, hydroxy, mercapto, amino optionally substituted by alkyl, carboxy, carbamoyl optionally substituted by alkyl, aminosulfonyl optionally substituted by a substituent selected from the group including alkyl, nitro, cyano, halogen and lower perfluoroalkyl, multiple degrees of substitution being allowed. Examples of "alkyl" as used herein include, but are not limited to, methyl, ethyl, n-butyl, n-pentyl, isobutyl, isopropyl and the like.

As used herein, the term "alkoxy" refers to the group RₐO-, where Rₐ is alkyl.

As used herein, the term "aryl" refers to a 6 to 10 carbon aromatic moiety. Examples of "aryl" as used herein include phenyl, pyridyl, pyrimidyl, pyridazyl, pyrazyl, and triazyl.

As used herein, the term "aryloxy" refers to the group RₐO-, where Rₐ is aryl.

As used herein the term "halogen" refers to the group fluorine, chlorine, bromine, and iodine.

As used herein the term "neoplasm" refers to an abnormal growth of cells or tissue and is understood to include benign, i.e., non-cancerous growths, and malignant, i.e., cancerous growths. The term "neoplastic" means of or related to a neoplasm.

As used herein the term "agent" is understood to mean a substance that produces a desired effect in a tissue, system, animal, mammal, human, or other subject Accordingly, the term "anti-neoplastic agent" is understood to mean a substance producing an anti-neoplastic effect in a tissue, system, animal, mammal, human, or other subject It is also to be understood that an "agent" may be a single compound or a combination or composition of two or more compounds.

As used herein, the term "solvate" is a complex of variable stoichiometry formed, by a solute and a solvent. Solvents, by way of example, include water, methanol, ethanol, or acetic acid.

As used herein the term "progenitor cells" refers to parent or precursor cells that are typically proliferating rapidly and give rise to cells that function in a tissue, system, animal, mammal, human, or other subject. Such progenitor cells include, bone marrow stem cells that give rise to erythrocytes, leukocytes and platelets; epithelial progenitors including epithelial stem cells of the intestinal tract, and hair follicle progenitors which give rise to hair.

As used herein the term "specific stage progenitor" refers to progenitors/precursors that can be assessed by a colony forming assay, e.g., CFU-GM an early (immature) granulocyte macrophage progenitor. As used herein the term "multiple stage progenitors" refers to a mixture of early and late (mature) progenitors/ precursors that can still divide (not terminally differentiated) and,produce daughter cells.

As used herein the term "phase specific anti-neoplastic compound" refers to anti-neoplastic compounds that are cell cycle specific. That is, the compounds act at or effect cells at specific phases of the cell cycle. For instance, there are about six dozen approved products for the treatment of malignancy. Many of the existing anti-neoplastic agents share a common mechanism of action and can be grouped together. These groups include alkylating agents such as melphalan, chlorambucil, cyclophosphamide, mechlorethamine, hexamethylmelamine, busulfan, carmustine, lomustine, and dacarbazine; antimetabolites such as 5-fluorouracil, methotrexate, cytarabine, mercaptopurine and thioguanine; antimitotic agents such as paclitaxel, vinblastine, vincristine; topoisomerase I inhibitors such as camptothecin and camptothecin derivatives; topoisomerase II inhibitors such as doxorubicin; and platinum coordination complexes such as cisplatin and carboplatin. These antineoplastic agents may either induce cytotoxic effects in a cell-cycle specific manner, i.e., are phase specific, or bind DNA and act in a non cell-cycle specific manner, i.e., are non-cell cycle specific (See Table 1).

As used herein, the term "effective amount" means that amount of a drug or pharmaceutical agent that will elicit the biological or medical response of a tissue, system, animal, mammal, human, or other subject that is being sought by a researcher or clinician. Furthermore the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder, and also includes amounts effective to enhance normal physiological function.

An anti-neoplastic agent that adversely affects normal progenitor cells is utilized in the present invention. Such adverse affects include myelosuppression, mucositis and alopecia.

Of special interest, are phase specific anti-neoplastic agents. Typical phase specific anti-neoplastic agents include diterpenoids, vinca alkaloids, epipodophyllotoxins, antimetabolites, and camptothecins.

Diterpenoids, which are derived from natural sources, are phase specific anti -cancer agents that operate at the G₂/M phases of the cell cycle. It is believed that the diterpenoids stabilize the β-tubulin subunit of the microtubules, by binding with this protein. Disassembly of the protein appears then to be inhibited with mitosis being arrested and cell death following. Examples of diterpenoids include, but are not limited to, paclitaxel and its analog docetaxel.

Paclitaxel, 5β,20-epoxy-1,2α,4,7β,10β,13α-hexa-hydroxytax-11-en-9-one 4,10-diacetate 2-benzoate 13-ester with (2R,3S)-N-benzoyl-3-phenylisoserine; is a natural diterpene-product isolated from the Pacific yew tree Taxus brevifolia and is commercially available as an injectable solution TAXOL™. It is a member of the taxane family of terpenes. It was first isolated in 1971 by Wani et al. J. Am. Chem, Soc., 93:2325. (1971), who characterized its structure by chemical and X-ray crystallographic methods. One mechanism for its activity relates to paclitaxel's capacity to bind tubulin, thereby inhibiting cancer cell growth. Schiff et al., Proc. Natl, Acad, Sci. USA, 77:1561-1565 (1980); Schiff et al., Nature, 277:665-667 (1979); Kumar, J. Biol, Chem, 256: 10435-10441 (1981). For a review of synthesis and anticancer activity of some paclitaxel derivatives see: D. G. I. Kingston *et al*., Studies in Organic Chemistry vol. 26, entitled "New trends in Natural Products Chemistry 1986", Attaur-Rahman, P.W. Le Quesne, Eds. (Elsevier, Amsterdam, 1986) pp 219-235.

Paclitaxel has been approved for clinical use in the treatment of refractory ovarian cancer in the United States (Markman et al., Yale Journal of Biology and Medicine, 64:583, 1991; McGuire et al., Ann. Intern, Med., 111:273,1989) and for the treatment of breast cancer (Holmes et al., J. Nat. Cancer Inst., 83:1797,1991.) It is a potential candidate for treatment of neoplasms in the skin (Einzig et. al., Proc. Am. Soc. Clin. Oncol., 20:46) and head and neck carcinomas (Forastire et. al., Sem. Oncol., 20:56, 1990). The compound also shows potential for the treatment of polycystic kidney disease (Woo et al., Nature, 368:750. 1994), lung cancer and malaria. Treatment of patients with paclitaxel results in bone marrow suppression (multiple cell lineages, Ignoff, R.J. et. al, Cancer Chemotherapy Pocket Guide 1998) related to the duration of dosing above a threshold concentration (50nM) (Kearns, C.M. et. al., Seminars in Oncology, 3(6) p.16-23, 1995).

Docetaxel, (2R,3S)- N-carboxy-3-phenylisoserine,N-*tert-*butyl ester, 13-ester with 5β-20-epoxy-1,2α,4,7β,10β,13α-hexahydroxytax-11-en-9-one 4-acetate 2-benzoate, trihydrate; is commercially available as an injectable solution as TAXOTERE™. Docetaxel is indicated for the treatment of breast cancer. Docetaxel is a semisynthetic derivative of paclitaxel *q.v.,* prepared using a natural precursor, 10-deacetyl-baccatin III, extracted from the needle of the European Yew tree. The dose limiting toxicity of docetaxel is neutropenia.

Vinca alkaloids are phase specific anti-neoplastic agents derived from the periwinkle plant. Vinca alkaloids act at the M phase (mitosis) of the cell cycle by binding specifically to tubulin. Consequently, the bound tubulin molecule is unable to polymerize into microtubules. Mitosis is believed to be arrested in metaphase with cell death following. Examples of vinca alkaloids include vinblastine, vincristine, and vinorelbine.

Vinblastine, vincaleukoblastine sulfate, is commercially available as VELBAN™ as an injectable solution. Although, it has possible indication as a second line therapy of various solid tumors, it is primarily indicated in the treatment of testicular cancer and various lymphomas including Hodgkin's Disease; lymphocytic and histiocytic lymphomas. Myelosuppression is the dose limiting side effect of vinblastine.

Vincristine, vincaleukoblastine, 22-oxo-, sulfate, is commercially available as ONCOVIN™ as an injectable solution. Vincristine is indicated for the treatment of acute leukemias and has also found use in treatment regimens for Hodgkin's and non-Hodgkin's malignant lymphomas. Alopecia and neurologic effects are the most common side effect of vincristine and to a lesser extent myelosupression and gastrointestinal mucositis effects occur.

Vinorelbine, 3',4'-didehydro -4'-deoxy-C'-norvincaleukoblastine [R-(R*,R*)-2,3-dihydroxybutanedioate (1:2)(salt)], commercially available as an injectable solution of vinorelbine tartrate (NAVELBINE™), is a semisynthetic vinca alkaloid. Vinorelbine is indicated as a single agent or in combination with other chemotherapeutic agents, such as cisplatin, in the treatment of various solid tumours, particularly non-small cell lung, advanced breast, and hormone refractory prostate cancers. Myelosuppression is the most common dose limiting side effect of vinorelbine.

Epipodophyllotoxins are phase specific anti-neoplastic agents derived from the mandrake plant. Epipodophyllotoxins typically affect cells in the S and G₂ phases of the cell cycle by forming a ternary complex with topoisomerase II and DNA causing DNA strand breaks. The strand breaks accumulate and cell death follows. Examples of epipodophyllotoxins include, but are not limited to, etoposide and teniposide.

Etoposide, 4'-demethyl-epipodophyllotoxin 9[4,6-0-(R)-ethylidene-β-D-glucopyranoside], is commercially available as an injectable solution or capsules as VePESID™ and is commonly known as VP-16. Etoposide is indicated as a single agent or in combination with other chemotherapy agents in the treatment of testicular and non-small cell lung cancers. Myelosuppression is the most common side effect of etoposide. The incidence of leucopenia tends to be more severe than thrombocytopenia.

Teniposide, 4'-demethyl-epipodophyllotoxin 9[4,6-0-(R)-thenylidene-β-D-glucopyranoside], is commercially available as an injectable solution as VUMON™ and is commonly known as VM-26. Teniposide is indicated as a single agent or in combination with other chemotherapy agents in the treatment of acute leukemia in children. Myelosuppression is the most common dose limiting side effect of teniposide. Teniposide can induce both leucopenia and thrombocytopenia.

Antimetabolite neoplastic agents are phase specific anti-neoplastic agents that act at S phase (DNA synthesis) of the cell cycle by inhibiting DNA synthesis or by inhibiting purine or pyrimidine base synthesis and thereby limiting DNA synthesis. Consequently, S phase does not proceed and cell death follows. Examples of antimetabolite anti-neoplistic agents include fluorouracil, methotrexate, cytarabine, mercaptopurine and thioguanine.

5-fluorouracil, 5-fluoro-2,4- (1H,3H) pyrimidinedione, is commercially available as fluorouracil. Adminsitration of 5-fluorouracil leads to inhibition of thymidylate synthesis and is also incorporated into both RNA and DNA. The result typically is cell death. 5-fluorouracil is indicated as a single agent or in combination with other chemotherapy agents in the treatment of carcinomas of the breast, colon, rectum, stomach and pancreas. Myelosuppression and mucositis are dose limiting side effects of 5-fluorouracil. Other fluoropyrimidine analogs include 5-fluoro deoxyuridine (floxuridine) and 5-fluorodeoxyuridine monophosphate.

Cytarabine, 4-amino-1-β-D-arabinofuranosyl-2 (1H)-pyrimidinone, is commercially available as CYTOSAR-U™ and is commonly known as Ara-C. It is believed that cytarabine exhibits cell phase specificity at S-phase by inhibiting DNA chain elongation by terminal incorporation of cytarabine into the growing DNA chain. Cytarabine is indicated as a single agent or in combination with other chemotherapy agents in the treatment of acute leukemia. Other cytidine analogs include 5-azacytidine and 2',2'-difluorodeoxycytidine (gemcitabine). Cytarabine induces leucopenia, thrombocytopenia, and mucositis.

Mercaptopurine, 1,7-dihydro-6H-purine-6-thione monohydrate, is commercially available as PURINETHOL™. Mercaptopurine exhibits cell phase specificity at S-phase by inhibiting DNA synthesis by an as of yet unspecified mechanism. Mercaptopurine is indicated as a single agent or in combination with other chemotherapy agents in the treatment of acute leukemia. Myelosuppresion and gastrointestinal mucositis are expected side effects of mercaptopurine at high doses. A useful mercaptopurine analog is azathioprine.

Thioguanine, 2-amino-1,7-dihydro-6H-purine-6-thione, is commercially available as TABLOID™. Thioguanine exhibits cell phase specificity at S-phase by inhibiting DNA synthesis by an as of yet unspecified mechanism. Thioguanine is indicated as a single agent or in combination with other chemotherapy agents in the treatment of acute leukemia. Myelosuppression, including leucopenia, thrombocytopenia, and anemia, is the most common dose limiting side effect of thioguanine administration. However, gastrointestinal side effects occur and can be dose limiting. Other purine analogs include pentostatin, erythrohydroxynonyladenine, fludarabine phosphate, and cladribine.

Methotrexate, N-[4[[(2,4-diamino-6-pteridinyl) methyl]methylamino] benzoyl]-L-gtutamic acid, is commercially available as methotrexate sodium. Methotrexate exhibits cell phase effects specifically at S-phase by inhibiting DNA synthesis, repair and/or replication through the inhibition of dyhydrofolic acid reductase which is required for synthesis of purine nucleotides and thymidylate. Methotrexate is indicated as a single agent or in combination with other chemotherapy agents in the treatment of choriocarconoma, meningeal leukemia, non-Hodgkin's lymphoma, and carcinomas of the breast, head, neck, ovary and bladder. Myelosuppression (leucopenia, thrombocytopenia, and anemia) and mucositis are expected side effect of methotrexate administration.

Camptothecins, including, camptothecin and camptothecin derivatives are Topoisomerase I inhibitors. Camptothecins cytotoxic activity is believed to be related to its Topoisomerase I inhibitory activity. Examples of camptothecins include topotecan, irinotecan and the various optical forms of 7-(4-methylpiperazino-methylene)-10,11-ethylenedioxy-24-camptothecin.

Topotecan HCl, (S)-10-[(dimethylamino)methyl]-4-ethyl,9-dihydroxy-1H-pyrano[3',4',6,7]indolizino[1,2-b]quinoline-3,14(4H,12H)-dione monohydrochloride, is commercially available as the injectable solution HYCAMTIN™. Topotecan is a semi-synthetic derivative of camptothecin which binds to the topoisomerase I - DNA complex and prevents religation of single strand breaks in DNA caused by topoisomerase I. It is believed that cytotoxicity occurs as a result of irreparable double strand breaks caused by interaction of the topoisomerase I : DNA : topotecan ternary complex with replication enzymes. Topotecan is indicated for the treatment of metastatic carcinoma of the ovary and small cell lung cancer. The dose limiting side effect of topotecan HCl is myelosuppression, primarily neutropenia.

Irinotecan HCl, (4S)-4,11-diethyl-hydroxy-9[(4-piperidinopiperidino) carbonyloxy]-1H-pyrano[3',4',6,7]indolizino[1,2-b]quinoline-3,14(4H,12H)-dione hydrochloride, is commercially available as the injectable solution CAMPTOSAR™. Irinotecan is a derivative of camptothecin which binds, along with its active metabolite SN-38, to the topoisomerase I - DNA complex. It is believed that cytotoxicity occurs as a result of irreparable double strand breaks caused by interaction of the topoisomerase I : DNA : irinotecan or SN-38 ternary complex with replication enzymes. Irinotecan is indicated for treatment of metastatic cancer of the colon or rectum. The dose limiting side effects of irinotecan HCl are myelosuppression, including neutropenia, and GI effects, including diarrhea.

Also of interest, is the camptothecin derivative of formula II following, currently under development, including the racemic mixture (R,S) form as well as the R and S enantiomers: 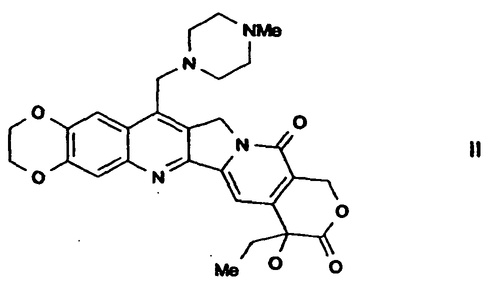 known by the chemical name "7-(4-methylpiperazino-methylene)-10,11-ethylenedioxy-20(R,S)-camptothecin (racemic mixture) or "7-(4-methylpiperazino-methylene)-10,11-ethylenedioxy-20(R)-camptothecin (R enantiomer) or "7-(4-methylpiperazino-methylene)-10,11-ethylenedioxy-20(S)-camptothecin (S enantiomer). Such compound as well as related compounds are described, including methods of making, in U.S. Patent Nos. 6,063,923; 5,342,947; 5,559,235; 5,491,237 and pending U.S. patent Application No. 08/977,217 filed November 24,1997.

In a preferred embodiment, the phase specific anti-neoplastic agent is paclitaxel. In an alternative preferred embodiment, the phase specific anti-neoplastic agent is topotecan.

As recited above, the combination of the present invention also includes a specific PDE4 inhibitor agent. PDE4 inhibitors are described, for instance, in "PDE4 inhibitors 1998°, Norman, Peter, Exp. Opin. Ther. Patents (1998) 8(7); and "Chronic Pulmonary inflammation and Other Therapeutic Applications of PDEIV Inhibitors", Stafford, Jeffrey A. and Feldman, Paul L., Ch. 8 Annual Reports In Medicinal Chemistry Ch. 8, 71-80 (1996).

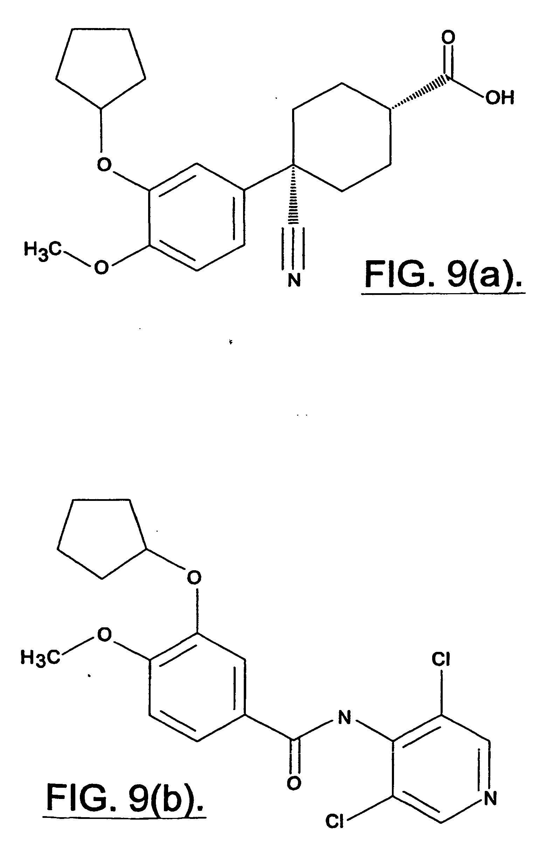

Of particular interest of the compounds of Formula I is the PDE4 inhibitor *cis-*4-cyano-4-[3-(cyclopentyloxy)-4-methoxyphenyl]cyclohexane-1-carboxylic acid or salt thereof. This compound has also been referred to as Ariflo™ and is depicted in Figure 9(a). The compound and related compounds as well as the synthesis of such compounds is described in U.S. Patent No. 5,643,946, said patent being incorporated herein by reference to extent that it teaches PDE4 inhibitor compounds and synthesis of the same.

Another PDE4 inhibitor that may be usefully employed in the present invention is piclamilast, i.e., N-(3,5-dichloropyrid-4-yl)-3-cyclopentyloxy-4-methoxybenzamide, depicted in Figure 9(b). Piclamilast and related compounds are described, as well as the synthesis thereof, in International Patent Application WO92/12961 and WO 97/48697.

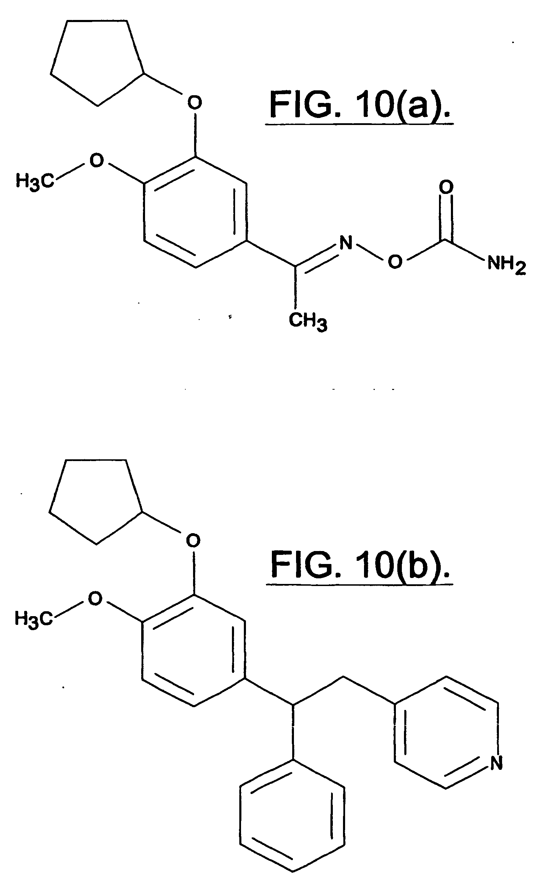

A further PDE4 inhibitor that can be usefully employed in the present invention is filaminast, 1-[3-(cyclopentyloxy)-4-methoxyphenyl] ethanone (E)-O-{aminocarbonyl}oxime, which is depicted in Figure 10(a). Filaminast and related compounds are described, as well as the synthesis thereof, in European Patent Application EP 470805A1.

Still another group PDE4 inhibitor compounds that can be usefully employed in the present invention is (+/-)-4-[2-(3-cyclopentyloxy-4-methoxyphenyl)-2-phenylethyl]pyridine (CDP-840), which is depicted in Figure 10(b). This compound is described, as well as the synthesis thereof, in International Patent Applications WO 94/14742; 97/23460; 97/23461; 97/38976; 97/42172; and 98/12178.

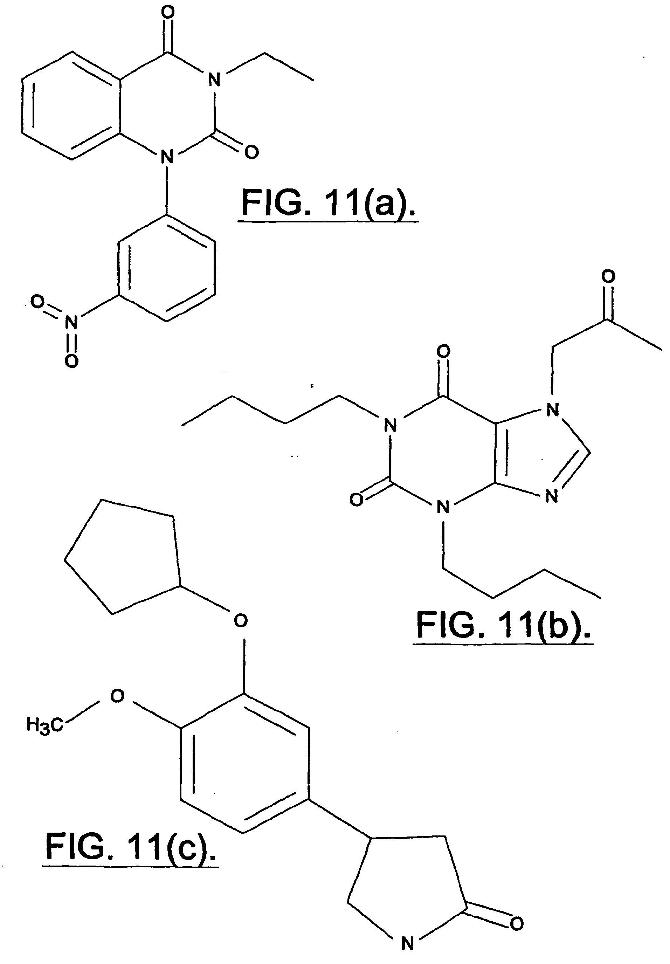

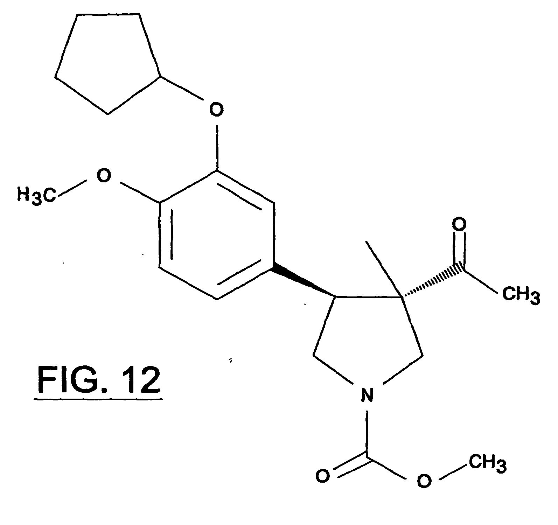

Additional PDE4 inhibitors which can be utilized in the present invention include:
(i) nitroquazone, depicted in Figure 11(a), and nitraquazone derivatives, such compounds being described in WO 93/07146 including the synthesis thereof;
(ii) denbufylline, i.e., 7-acetonyl,1,3dibutytxanthine, made by SmithKline Beecham and depicted in Figure 11(b);
(iii) rolipram, depicted in Figure 11(c);
   RS-25344 [8-aza-1-(3-nitrophenyl)-3-(4-pyridylmethyl)-2,4-quinazolinedione], and related compounds, such compounds being described in WO 93/07146 and 93/19068 including the synthesis thereof;
(v) CP-77059 1-(3-carbomethoxyphenyl)-3-benzylpyrido[2,3d]pyrimidine-2,4(1H,3H)dione and related compounds, such compounds being described in WO 96/40636 and 97/05105 including the synthesis thereof; and
(vi) GI 193600X [methyl (3S,4S)-3-acetyl-4-[3(cyclopentyloxy)-4-(methyloxy)phenyl]-3-methyl-1-pyrrolidinecarboxylate] available from Glaxo Wellcome, Inc., depicted in Figure 12.

In one embodiment, the PDE4 inhibitor is *cis*-4-cyano-4-[3-(cyclopentyloxy)-4-methoxyphenyl]cyclohexane-1-carboxyclic acid or salt thereof; N-(3,5-dichloropyrid-4-yl)-3-cyclopentyloxy-4-methoxybenzamide; 1-[3-(cyclopentyloxy)-4-methoxyphenyl] ethanone (E)-O-{aminocarbonyl}oxime: or (+/-)-4-[2-(3-cyclopentyloxy-4-methoxyphenyl)-2-phenylethyl]pyridine.

Rolipram, a PDE4 inhibitor agent, when used alone had little protective effect in the cell based assays utilized herein. This is in agreement with the lack of effect of rolipram when used alone on various neutrophil functions (Au B-T et al. British J Pharmacol 123: 1260-1266, (1998); Nielsen et al. J Allergy Clin Immunol, 86: 801-808, (1990)). However, a significant synergism between PDE4 inhibitors and agents that activate adenylate cyclase and raise intracellular levels of cAMP, e.g. forskolin was observed (Nicholson, CD; Shahid, M. Pulm Pharmacol, 7:1-17 (1994); Au B-T et al. British J Pharmacol, 123: 1260-1266, (1998); Nielsen et al. J Allergy Clin Immunol, 86: 801-808, (1990)). While not wishing to be bound by theory, the present inventors believe that in human cancer patients an activator of adenylate cyclase may not be needed because serum levels of the adenylate cyclase activator prostaglandin E2 (PGE₂), in these patients are elevated (Young MR, Cancer Met. Rev., 13: 337-348 (1994); Uotila P., Cancer Immunol Immunother, 43: 1-9, (1996); Baxevanis CN et al. Eur J Cancer, 33 (8): 1202-1208, (1997)), thus contributing to elevated cAMP levels.

The present invention further includes an agent effective in raising intracellular concentrations of cAMP or analogs thereof along with the phase specific cancer agent and the PDE4 inhibitor agent. Suitable compounds include agents which (1) increase cAMP levels by activating surface receptors which are Gₛ protein coupled to the cAMP generating enzyme adenylyl cyclase1; and (2) increase cAMP levels by directly stimulating adenylate cyclase, including, forskolin, and stable analogs of cAMP including, dibutyryl cAMP; 8-bromo c-AMP.

In one embodiment, the agent, which is effective to raise intracellular levels of CAMP, is selected from terbutaline, albuterol, pirbuterol, bitolterol, metaproterenol, formoterol, and salmeterol or salt thereof.

Terbutaline, (+/-)-α[(tert-butylamino)methyl]-3,5-dihydrobenzyl alcohol sulfate (2:1 salt), is commercially available as BRETHINE™. Terbutaline is described in U.S. Patent 3,938,838. Albuterol, racemic α¹[(tert-butylamino) methyl]4-hydroxy-m-xylene-α,α'-diol sulfate (2:1 salt), is commercially available as PROVENTIL™. Pirbuterol, (R,S) α⁶-([(1,1-dimethylethyl)amino]methyl)-3-hydroxy-2,6-pyridinedimethanolmonoacetate salt, is commercially available as MAXAIR™. Bitolterol, 4-[2-{(1,1-dimethylethyl)amino}-1-hydroxyethyl]-1,2-phenylene-4-methylbenzoate methanesulfonate salt, is commercially available as TORNALATE™. Metaproterenol, 1-(3,5-dihydroxyphenyl)-2-isopropylaminoethanol sulfate, is commercially available as ALUPENT™. Metaproterenol is described in U.S. patent 3,341,594. Formoterol, [(R*,R*)-(+/-)-N-[2-hydroxy-5-[1-hydroxy-2[[2-(4-methoxyphenyl)-1-methylethyl]ethyl]phenyl]formamide], is described in U.S. patent 3,994,974.

Salmeterol, 4-hydroxy-α1[((6-(4-phenylbutoxy)hexyl)amino) methyl]-1,3-benzenedimethanol, preferably the 1-hydroxy-2-napthalenecarboxylate salt (xinasoate), is commercially available as SEREVENT™ and is described in GB 2140800.

In another embodiment, the phase specific anti-neoplastic agent is paclitaxel, the PDE4 inhibitor is *cis*-4-cyano-4-[3-(cyclopentyloxy)-4-methoxyphenyl] cyclohexane-1-carboxylic acid or salt thereof and the agent effective to raise intracellular levels of cAMP is salmeterol or salt thereof.

In an alternative embodiment, the phase specific anti-neoplastic agent is topotecan, the PDE4 inhibitor is *cis*-4-cyano-4-[3-(cyclopentyloxy)-4-methoxyphenyl cyclohexane-1-carboxylic acid or salt thereof and the agent effective to raise intracellular levels of CAMP is salmeterol or salt thereof.

The animal requiring treatment using the combination of the present Invention is a mammal, such as a human being.

When utilized, the agent, which is effective to raise intracellular cAMP concentrations is administered concomitantly with the PDE4 inhibitor compound and before or after administration of the anti-neoplastic agent. Alternatively, the agent, which is effective to raise intracellular cAMP concentrations is administered concomitantly with the PDE4 inhibitor compound and the anti-neoplastic agent Alternatively, the agent which is effective to raise intracellular cAMP concentrations is administration sequentially with the phase specific anti-neoplastic agent and the PDE4 inhibitor agent in any sequence and within any time period that is suitable to achieve the desired therapeutic effect. The PDE4 inhibitor agent and the agent which is effective to raise intracellular cAMP concentrations are administered in a manner with the phase specific anti-neoplastic agent, so as to achieve a preventative or ameliorative effect on side effects that would otherwise be present in the absence of the PDE4 inhibitor agent and the agent which is effective to raise intracellular cAMP concentrations.

The anti-neoplastic agents. PDE4 inhibitor agents and agents which are effective to raise intracellular cAMP concentrations of the present invention may be administered by any appropriate route. Suitable routes include oral, rectal, nasal, topical (including buccal and sublingual), vaginal, and parenteral (including subcutaneous, intramuscular, intraveneous, intradermal, intrathecal, and epidural). It will be appreciated that the preferred route may vary with, for example, the condition of the recipient of the combination. It will also be appreciated that each of the agents/activators administered may be administered by the same or different routes and that one or more of the phase specific anti-neoplastic agent, PDE4 inhibition agent and adenylate cyclase inhibitor may be compounded together in a pharmaceutical composition/formulation.

The present invention may also be employed with other cancer treatments. In particular, in anti-neoplastic therapy, combination therapy with other chemotherapeutic, hormonal, antibody agents as well as surgical and/or radiation treatments other than those mentioned above are envisaged. Combination therapies of the invention thus include the administration of at least one anti-neoplastic agent and at least one PDE4 inhibitor agent and at least one agent which is effective to raise intracellular cAMP concentrations as well as optional use of other therapeutic agents including other anti-neoplastic agents. Such combination of agents may be administered together or separately and, when administered separately this may occur simultaneously or sequentially in any order, both close and remote in time. The amounts of the compound(s) and the other pharmaceutically active agent(s) and the relative timings of administration will be selected in order to achieve the desired combined therapeutic effect.

Pharmaceutical formulations adapted for oral administration may be presented as discrete units such as capsules or tablets; powders or granules; solutions or suspensions in aqueous or non-aqueous liquids; edible foams or whips; or oil-in-water liquid emulsions or water-in-oil liquid emulsions.

For instance, for oral administration in the form of a tablet or capsule, the active drug component can be combined with an oral, non-toxic pharmaceutically acceptable inert carrier such as ethanol, glycerol, water and the like. Powders are prepared by comminuting the compound to a suitable fine size and mixing with a similarly comminuted pharmaceutical carrier such as an edible carbohydrate, as, for example, starch or mannitol. Flavoring, preservative, dispersing and coloring agent can also be present.

Capsules are made by preparing a powder mixture as described above, and filling formed gelatin sheaths. Glidants and lubricants such as colloidal silica, talc, magnesium stearate, calcium stearate or solid polyethylene glycol can be added to the powder mixture before the filling operation. A disintegrating or solubilizing agent such as agar-agar, calcium carbonate or sodium carbonate can also be added to improve the availability of the medicament when the capsule is ingested.

Moreover, when desired or necessary, suitable binders, lubricants, disintegrating agents and coloring agents can also be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrators include starch, methyl cellulose, agar, bentonite, xanthan gum Tablets are formulated, for example, by preparing a powder mixture, granulating or slugging, adding a lubricant and disintegrant and pressing into tablets. A powder mixture is prepared by mixing the compound, suitably comminuted, with a diluent or base as described above, and optionally, with a binder such as carboxymethylcellulose, an alginate, gelatin, or polyvinyl pyrrolidone, a solution retardant such as paraffin, a resorption accelerator such as a quaternary salt and/or an absorption agent such as bentonite, kaolin or dicalcium phosphate. The powder mixture can be granulated by wetting with a binder such as syrup, starch paste, acacia mucilage or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc or mineral oil. The lubricated mixture is then compressed into tablets. The compounds of the present invention can also be combined with free flowing inert carrier and compressed into tablets directly without going through the granulating or slugging steps. A dear or opaque protective coating consisting of a sealing coat of shellac, a coating of sugar or polymeric material and a polish coating of wax can be provided. Dyestuffs can be added to these coatings to distinguish different unit dosages.

Oral fluids such as solution, syrups and elixirs can be prepared in dosage unit form so that a given quantity contains a predetermined amount of the compound. Syrups can be prepared by dissolving the compound in a suitably flavored aqueous solution, while elixirs are prepared through the use of a non-toxic alcoholic vehicle. Suspensions can be formulated by dispersing the compound in a non-toxic vehicle. Solubilizers and emulsifiers such as ethoxylated isostearyl alcohols and polyoxy ethylene sorbitol ethers, preservatives, flavor additive such as peppermint oil or natural sweeteners or saccharin or other artificial sweeteners can also be added.

Where appropriate, dosage unit formulations for oral administration can be microencapsulated. The formulation can also be prepared to prolong or sustain the release as for example by coating or embedding particulate material in polymers, wax or the like.

The agents according to the present invention can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

Agents according to the present invention may also be delivered by the use of monoclonal antibodies as individual carriers to which the compound molecules are coupled. The compounds may also be coupled with soluble polymers as targetable drug carriers. Such polymers can include polyvinylpyrrolidone, pyran copolymer, polyhydroxypropylmethacrylamide-phenol, polyhydroxyethylaspartamidephenol, or polyethyleneoxidepolylysine substituted with palmitoyl residues. Furthermore, the compounds may be coupled to a class of biodegradable polymers useful in achieving controlled release of a drug, for example, polylactic acid, polyepsilon caprolactone, polyhydroxy butyric acid, polyorthoesters, polyacetals, polydihydropyrans, polycyanoacrylates and cross-linked or amphipathic block copolymers of hydrogels.

Pharmaceutical formulations adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3(6), 318 (1986).

Pharmaceutical formulations adapted for topical administration may be formulated as ointments, creams, suspensions, lotions, powders, solutions, pastes, gels, sprays, aerosols or oils.

For treatments of the eye or other external tissues, for example mouth and skin, the formulations are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base.

Pharmaceutical formulations adapted for topical administrations to the eye include eye drops wherein the active ingredient is dissolved or suspended in a suitable carrier, especially an aqueous solvent

Pharmaceutical formulations adapted for topical administration in the mouth include lozenges, pastilles and mouth washes.

Pharmaceutical formulations adapted for rectal administration may be presented as suppositories or as enemas.

Pharmaceutical formulations adapted for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as a nasal spray or as nasal drops, include aqueous or oil solutions of the active ingredient.

Pharmaceutical formulations adapted for administration by inhalation include fine particle dusts or mists that may be generated by means of various types of metered dose pressurised aerosols, nebulizers or insufflators.

Pharmaceutical formulations adapted for vaginal administration may be presented as pessaries, tampons, creams, gels, pastes, foams or spray formulations.

Pharmaceutical formulations adapted for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets.

It should be understood that in addition to the ingredients particularly mentioned above, the formulations may include other agents conventional in the art having regard to the type of formulation in question, for example those suitable for oral administration may include flavoring agents.

Salts encompassed within the term "pharmaceutically acceptable salts" refer to non-toxic salts of the compounds of this invention which are generally prepared by reacting a free base with a suitable organic or inorganic acid or by reacting the acid with a suitable organic or inorganic base.

The pharmaceutical combination includes an anti-neoplastic agent, a PDE4 inhibitor, and an agent, which is effective to raise intracellular cAMP concentrations. The anti-neoplastic agent, PDE4 inhibitor, and agent that is effective to raise intracellular cAMP concentrations are as described above. In another embodiment, the phase specific anti-neoplastic agent is paclitaxel, the PDE4 inhibitor is *cis*-4-cyano-4-[3(cyclopentyloxy)-4-methoxyphenyl]cyclohexane-1-carboxylic acid or salt thereof and if included the agent effective to raise intracellular levels of cAMP is salmeterol or salt thereof. In still another embodiment, the phase specific anti-neoplastic agent is topotecan, the PDE4 inhibitor is *cis*-4-cyano-4-[3-(cyclopentyloxy)-4-methoxyphenyl]cyclohexane-1-carboxylic acid or salt thereof and if included the agent effective to raise intracellular levels of cAMP is salmeterol or salt thereof.

The present invention provides a cancer treatment combination whereby detrimental side effects associated with anti-neoplastic agents that adversely affect normal progenitor cells are attenuated. It will be understood by those skilled in the art that the specific cancers treated will depend on the specific anti-neoplastic agents used. As such the expected side effects as well may vary depending on the dose, routes, and cycles of anti-neoplastic agents utilized to treat a specific tumor. Typical, side effects which may be attentuated include but are not limited to myelosuppression including neutropenia, thrombocytopenia, or anemia; mucositis; and alopecia. In a preferred embodiment, the side effect attentuated is myelosuppression or mucositis. In a more preferred embodiment, the side effect attentuated is neutropenia.

As indicated, therapeutically effective amounts of the specific anti-neoplastic agent, the PDE4 inhibitor, and the agent that is effective to raise intracellular cAMP concentrations, are administered to a mammal. Typically, the therapeutically effective amount of one of the agents of the present invention will depend upon a number of factors including, for example, the age and weight of the mammal, the precise condition requiring treatment, the severity of the condition, the nature of the formulation, and the route of administration. Ultimately, the therapeutically effective amount will be at the discretion of the attendant physician or veterinarian.

Typically, the anti-neoplastic agent will be given in the range of 0.1 to 100 mg/kg body weight of recipient (mammal) per day and more usually in the range of 1 to 10 mg/kg body weight per day. Acceptable daily dosages of the PDE4 inhibitor for preventing/reducing the severity of side effects induced by administration of a phase specific anti-neoplastic agent, may be from about 0.1 to about 1000 mg/day, and preferably from about 0.2 to about 100 mg/day. Acceptable daily dosages of the agent which is effective to raise intracellular cAMP concentrations for stimulating cAMP production to assist in reducing the severity of side effects induced by administration of a anti-neoplastic agent, may also be from about 0.1 to about 1000 mg/day, and preferably from about 0.2 to about 100 mg/day.

In the examples following as well as in other portions of the specification following abbreviations are used
- CAMP: cyclic adenosine monophosphate
- CELISA: cell-based enzyme linked immunoabsorbent assay
- CFU-GM: colony forming unit-granulocyte macrophages
- DMEM: Dulbecco's Modified Eagle's Media
- DMSO: dimethyl sulfoxide
- ELISA: enzyme linked immunoabsorbent assay
- EDTA: ethylenediaminetetraacetic acid
- FACS: fluorescent-activated cell analysis
- FBS: fetal bovine serum
- G-CSF: granulocyte-colony stimulating factor
- GM-CSF: granulocyte-macrophage-colony stimulating factor
- HBSS: Hank's Balanced Salt Solution
- IMDM: Iscove's Modified Dulbecco's medium
- PBS: phosphate buffered saline
- PDE4: phosphodiesterase 4
- RT: room temperature
- SRB: sulforhodamine B

### Example 1

### Preparation of PDE4 Stock Solutions.

PDE4 inhibitors were synthesized utilizing published procedures. The PDE4 inhibitors were prepared as 1.0 mM stock solutions in DMSO. The PDE4 inhibitors prepared were Rolipram (Baures, PW, 1993 J.Med. Chem. 36(3274-3277.); Ariflo™ (SB-207499, Christensen S.B. 1998 *J Med* Chem 41, 821-835); GI193600X (Stafford JA et al., Journal of Medicinal Chemistry 38(26): 4972-4975, (1995)); RP-73401 (RPR Patent, WO9748697); CDP-840 (Merck, Celltech patents WO9723460, WO9723461, WO9738976, WO9742172, WO9812178); nitraquazone and denbufylline (Norman P., 1998, Exp. Opin. Ther. Patents, 8 (7)), 771-784); GI198425X (Syntex patents, WO-9307146, WO-9319068); and GI147404X (Pfizer Inc., WO-9640636. WO-9705105). The PDE4 stock solutions were utilized in the following examples.

Prevention of myelosuppression was shown by protection of neutrophil progenitors by PDE4 inhibitors from the cytotoxic effects of cancer chemotherapeutic agents. In Examples 2-7 a variety of chemically distinct PDE4 inhibitors were tested for their ability to protect neutrophil progenitors. Three methods, more completely described in the following examples, were utilized for a determination of cell survival and ability to produce daughter cells of primary human neutrophil progenitors: (1) a FACS assay which determined the percent of live cells by size and granularity; (2) a colony forming assay which measured the number of CFU-GM progenitors (a specific early stage neutrophil progenitor); and (3) a cell-based EUSA which measured CD15 antigen the value of which was proportional to the number of neutrophil lineage cells. This assay allows evaluation of neutrophil progenitors (cells that can divide and produce daughter neutrophil-lineage cells) at multiple stages of differentiation.

### Example 2

### Flow cytometric evaluation of expanded CD34⁺ cells treated with rolipram plus forskolin followed by treatment with paclitaxel.

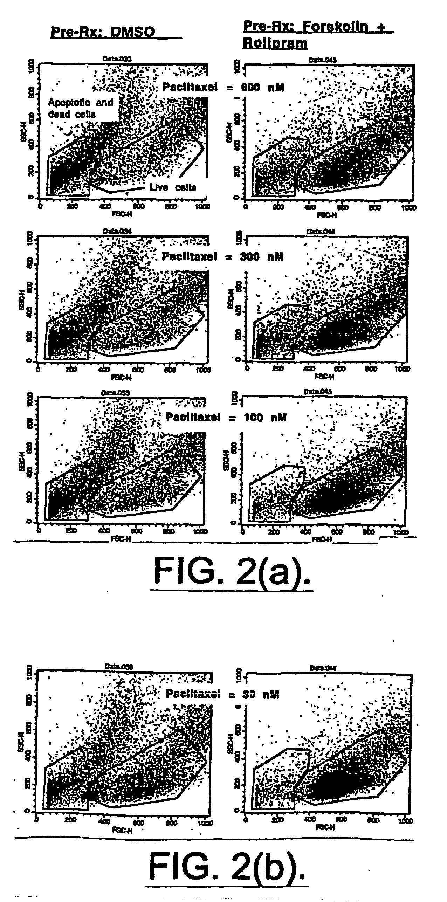

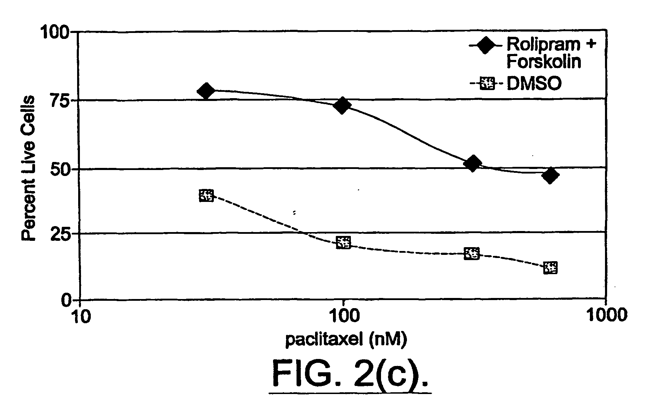

Human bone marrow samples were collected from presumed healthy human donors following informed consent through an agreement with Duke University Medical Center, Durham, North Carolina. Mononuclear cells were isolated using a 1.077 Ficoll-Paque gradient according to the published procedure (Dornsife, RE et al., Antimicrob Agents Chemother, 35: 322-328, (1991)) and were suspended in HBSS with 2% FBS at a concentration of 5 X 10⁷ cells/ml. CD34⁺ stem cells were isolated by "StemSep™ CD34⁺ stem cell selection kit" by following the instructions from the manufacturer (Stem Cell Technologies). Cells were adjusted to 1 x 10⁶ cells/ml in complete growth media and maintained in a humidified, 5% CO₂ incubator. The complete growth media was IMDM supplemented with 20% FBS, 1% penicillin-streptomycin antibiotics, the recombinant human cytokines (Biosource)Flt-3/Flk-2 ligand (50 ng/ml), stem cell factor (50 ng/ml), interleukin-3 (40ng/ml), and G-CSF (10 ng/ml). The cells were maintained at a density of between 0.5 and 3 x 10⁵ cells/ml. The CD34+ stem cells were expanded in culture over an average range of 11 to 23 days. Five groups of expanded CD34+ cells were treated with rolipram (1µM), plus Forskolin (100 µM), an adenylate cyclase activator, for 24 hours and five groups of expanded CD34+ cells were treated with DMSO for 24 hours. After 24 hours, to the 5 sets of cells that were treated with DMSO and 5 groups of cells treated with rolipram plus forskolin, paclitaxel was added to give final concentrations of 0, 30, 100, 300, and 600 nM of paclitaxel respectively and incubated for an additional 24 hours. After the 48 hours of treatment, the cells were counted, washed and re-suspended in PBS with 2% FBS. A total of 1 x 10⁶ cells were pelleted and then fixed with 0.5% paraformaldeyde in PBS + 2% FBS solution. The prepared cells were analyzed on a BD FACSort flow cytometer. The results are depicted in Figure 2.

The expanded CD34⁺ stem cells treated with rolipram plus forskolin prior to paclitaxel treatment, in Example 2, were analyzed on a flow cytometer (FACS) for surviving and apoptotic cell fractions (Fig. 2). The pretreatment with rolipram plus forskolin significantly reduced the apoptotic fraction in paclitaxel treated cells when compared with the cells pretreated with only DMSO. Conversely, the surviving cell fraction was much higher in cells pretreated with the chemoprotective regimen (Fig. 2). The surviving cell fractions after the pretreatment increased from 45% to 86% 27% to 81%, 23% to 63%, and 18% to 59% at 30nM, 100nM, 300nM, and 600nM of paclitaxel, respectively.

A T-test analysis (One-way ANOVA, with application of either equal or unequal variance [difference in standard error of the mean (sem) < or > 3-fold, respectively] was performed to compare between treatment groups to determine if statistically significant differences existed (MS Excel, SAS JMP). The pretreatment data (rolipram plus forskolin) were determined to be statistically significantly different from DMSO controls (p = 0.0062), indicating that more cells survived.

It is important to note that the expanded CD34⁺ stem cells could contain, in addition to the progenitors of neutrophils, the progenitors of platelets, erythrocytes, and/or lymphocytes (B, T, and NK). The protection of the expanded CD34⁺ cells from the cytotoxic effects of paclitaxel by the rolipram plus forskolin pretreatment suggests that the PDE4 inhibitors can potentially protect cancer patients from multiple blood lineage hematopoietic toxicities (neutropenia, thrombocytopenia, and anemia).

### Example 3

### Protection of specific early neutrophil progenitors (CFU-GMs) as measured by colony forming assay - rolipram plus forskolin followed by treatment with paclitaxel, 5-fluorouracil, or etoposide.

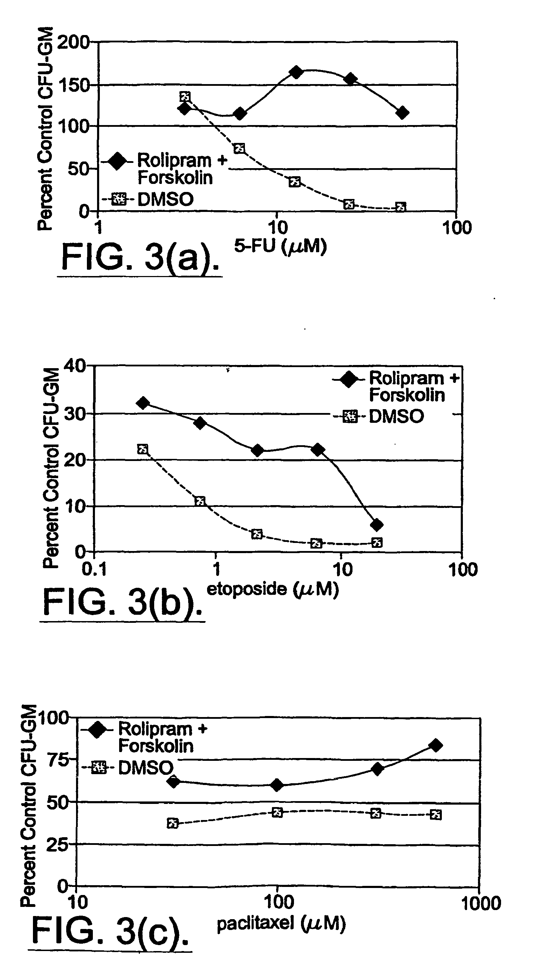

Expanded CD34⁺ cells, prepared according to the procedure of Example 2, were isolated and pretreated with DMSO or rolipram plus forskolin as described in Example 2. After 24 hours, varying amounts of paclitaxel, 5-fluorouracil, and etoposide were added and incubated for an additional 24 hours. After the 48 hours of treatment, the cells were counted, washed twice with 4 ml of HBSS supplemented with 2% FBS, and resuspended in complete growth media. The cells were then plated in a semi solid growth medium for the growth of CFU-GMs following a published method with the exception that the G-CSF (10ng/ml) was included in place of GM-CSF and erythropoietin (Dornsife, RE et al., Antimicrob Agents Chemother, 35: 322-328, (1991); Dornsife RE; Averett DR., Antimicrob Agents Chemother, 40(2): 514-519, (1996)). The CFU-GMs were counted after 12 to 14 days. The results are depicted in Figure 3.

Cancer patients are particularly predisposed to bacterial infection due to the fact that they are usually immunosuppressed by both the cancer and anti-neoplastic treatments. Patients with neutrophil counts of 500/µl have a substantially increased susceptibility for infection. Neutrophil counts of even less confers an escalating risk as the neutrophil count drops.

To examine the potential to protect specific early neutrophil progenitors (CFU-GM) from the cytotoxic effects of anti-neoplastic drugs a colony forming assay (Fig. 3) was used after the cells were exposed for 48 hours of treatments in the previously specified liquid culture in Example 3. After the expanded CD34⁺ cells were treated with varying concentrations of 5-fluorouracil or etoposide the number of CFU-GM colonies decreased in a dose dependent manner with increasing amounts of the drug (Fig. 3(a) εt 3(b)). In contrast, expanded CD34⁺ cells treated with paclitaxel did not exhibit a good dose response (Fig. 3(c)). However, a pretreatment of the expanded CD34⁺cells with rolipram, and forskolin, increased the survival of CFU-GM progenitors, as the number of resultant colonies were increased to a statistically significant level (95% confidence) in cells pretreated with the chemoprotective regimen prior to a treatment with 5-fluorouracil (p = 0.014), etoposide (p = 0.046), or paclitaxel (p = 0.012) when compared with cells pretreated with only DMSO (Fig. 3).

### Example 4

### Protection of multiple stage neutrophil progenitors as measured by CD15 neutrophil antigen cell-based ELISA (CELISA) method - rolipram plus forskolin followed by treatment with paclitaxel, 5-fluorouracil, etoposide, vinblastine, vinorelbine, methotrexate, or topotecan.

Expanded CD34⁺ stem cells were isolated as described in Example 2 and grown in complete growth media. The expanded CD34+ cells were treated with DMSO or rolipram plus forskolin as previously described. After 24h, varying amounts of paclitaxel, 5-fluorouracil, etoposide, vinblastine, vinorelbine, methotrexate, or topotecan were added and incubated for an additional 24 hours. After the treatment, the cells were counted, washed twice with 4 ml of HBSS supplemented with 2% FBS, and resuspended in complete growth media. The expression of CD15 antigen was used as a measure of the number of neutrophil lineage cells expanded from surviving progenitors of multiple stages of differentiation. (modified version of assay described in Warren et al., 1995 Stem Cells 13:167-174).

The cells were plated at a set number into 96-well plates In 100 µl volume per well of complete media. In some experiments, an additional 100 µl of complete media was added to plates maintained for later time points before harvest. The plates were harvested at different time points by centrifuging at 450 G for 10 minutes at room temperature (RT) and the cells were washed several twice in HBSS by centrifugation of the plates. The cells were removed by suspension twice in HBSS with 5 mM EDTA with 100 µl per well and transfer of the total 200 µl volume to capture plates (Costar DNA-bind, #2498) that had been pretreated with 1% gluteraldehyde and stored (Warren et al., 1995 Stem Cells 13:167-174).

For CELISA, the preservative solution (PBS, 0.1%BSA, 0.1% azide) was removed from the capture plate and the wells were washed with 100 µl PBS. A mouse anti-human CD15 antibody (Biosource, Clone B-H8 0.5 µg/well, 100 µl PBS with 5% goat sera) was added and incubated at RT for 60 min. The plates were washed twice with 300 µl PBS/well and incubated with a secondary antibody (goat anti-mouse IgM-biotin, Sigma B-9265, 0.105 µg/well, 100 µl PBS with 5% goat sera) at RT for 60 min. After three to six 200 µl/well washes, strepavidin-horseradish peroxidase (Pierce 21126, 0.1 µg/well in a 1% solution of stable peroxide buffer, Pierce 34062) was added. The plates were incubated for ten minutes and washed three to six times with 200 µl/well. For the final detection step, the chemiluminescent reagent Supersignal™ Ultra (Pierce 34075) prepared just prior to use by mixing equal parts of the luminol enhancer and the stable peroxide was added to the plates (100 µl per well) and gently mixed for five minutes at RT. The plates were read on a Wallace Victor II machine for one second for chemiluminescence at 425 nm.

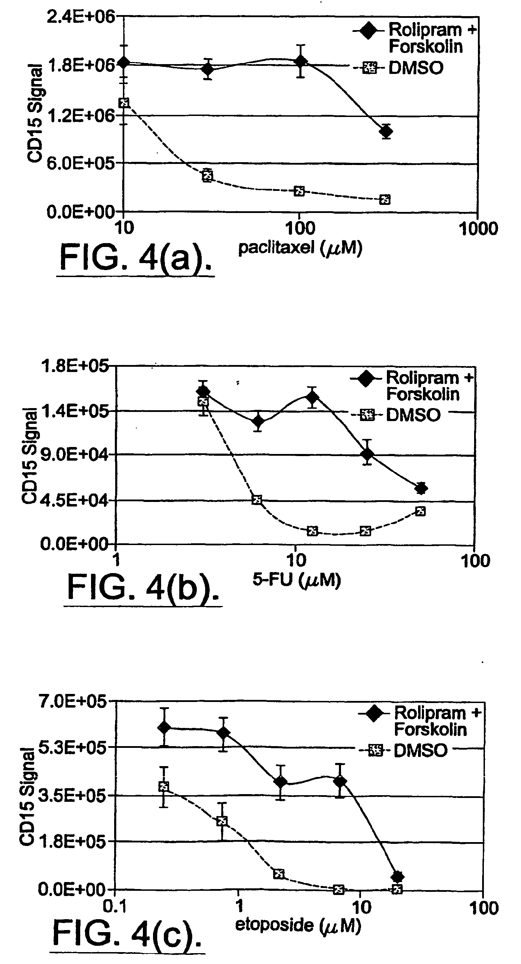

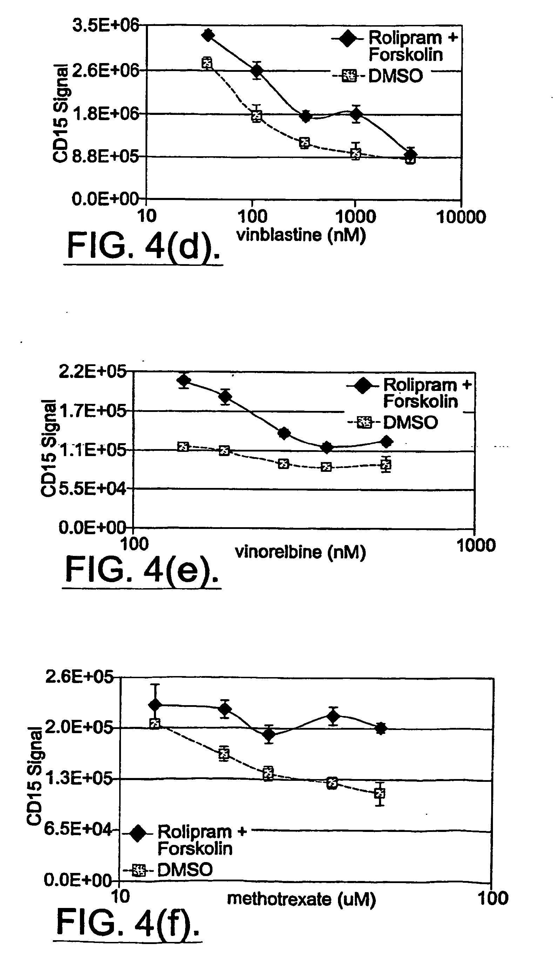

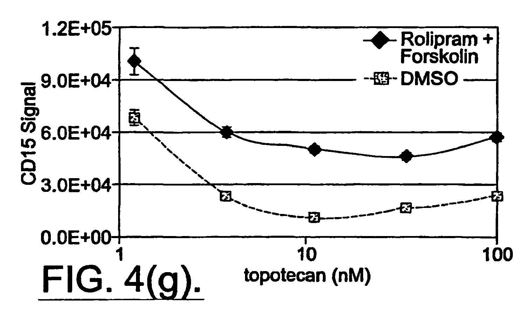

The data from the Wallac Victor were transferred to Excel for analysis. Averages of replicate wells from treated cells were calculated along with standard error of the mean (sem) of the counts minus the background signal from replicate wells with media only (no cells). The results are depicted in Figure 4.

The protection of neutrophil progenitors from the cytotoxic effects of anti-neoplastic agents by pretreatment with rolipram in combination with forskolin was further confirmed in Example 4 by a CELISA method (Fig. 4). The expression of CD15, an antigen expressed on stage specific CFU-GM progenitors as well as other granulocytic (neutrophil) lineage cells, by CELISA was used to confirm the statistically significant protection of multiple stages of neutrophil progenitors. This assay measures the recovery and expansion of CD15+ cells from neutrophil progenitors (those able to divide and produce CD15+ daughter cells). The CD15 signal from the cell cultures decreased with increasing concentrations of paclitaxel, 5-fluorouracil, etoposide, vinblastine, vinorelbine, methotrexate, or topotecan (Fig. 4A-4G). A pretreatment of the expanded CD34⁺ cells with rolipram plus forskolin increased the CD15 signal antigen to a statistically significant level (99% confidence) (paclitaxel p = 1.3 X10⁻¹¹, 5-FU p = 4.8 x 10⁻⁷, etoposide p = 5.8 X 10⁻⁶, vinblastine p = 4.4 X 10⁻³, vinorelbine p = 1.5 X 10⁻⁹, methotrexate p = 1.2 X 10⁻⁷, and topotecan p = 9.8 X 10⁻⁸), indicating that more progenitors survived the cytotoxic effects of the anti-neoplastic agents to divide and produce more CD15+ daughter cells versus progenitors pretreated with only DMSO (Fig. 4).

### Example 5

### Protection of multiple stage neutrophil progenitors as measured by CD15 neutrophil antigen-based CELISA method -PDE4 inhibitors (other than rolipram) plus forskolin followed by treatment with paclitaxel

Neutrophil progenitors were protected by PDE4 Inhibitors (a) Ariflo™, or (b) Piclamilast, (c) CDP-840 [GR259653X], (d)) CDP-840 [GR259654X], (e) Nitraquazone, (f) Denbufylline, (g) RS-25344, (h) CP-77059, and (i) GI193600X each in combination with forskolin from the cytotoxic effects of paclitaxel as measured by CELISA. Experimental details of this experiment are the same as described under Example 4 except that another PDE4 inhibitor (1 µM) was substituted for rolipram. The results are depicted in Figure 5.

Example 5 illustrates the protection of neutrophil progenitors from the cytotoxic effects of paclitaxel by a variety of PDE4 specific inhibitors each in combination with forskolin as measured by the CEUSA method. Pretreatment of expanded CD34+ cells with the different PDE4 inhibitors each plus forskolin increased expression of CD15+ signal to a statistically significant level (Ariflo™ p = 2.7 X 10⁻⁹, Piclamilast p = 8.5 X 10⁻¹⁵, CDP-840 [GR259653X] p = 1.9 X 10⁻⁵ CDP-840 [GR259654X] p = 6.5 X 10⁻¹³, Nitraquazone p = 1.0 X 10⁻⁸. Denbufylline p = 1.1 X 10⁻⁹, RS-25344 p = 2,2 X 10⁻⁶, CP-77059 p = 3.2 X 10⁻⁷, and GI193600X p = 3.4 X 10⁻¹³), suggesting that more neutrophil progenitors survived the cytotoxic effects of the anti-neoplastic agent paclitaxel (Fig. 5(a)- Fig. 5(i)).

### Example 6

### Protection of neutrophil progenitors as measured by CD15 neutrophil antigen CELISA method-Ariflo™ plus forskolin followed by treatment with topotecan.

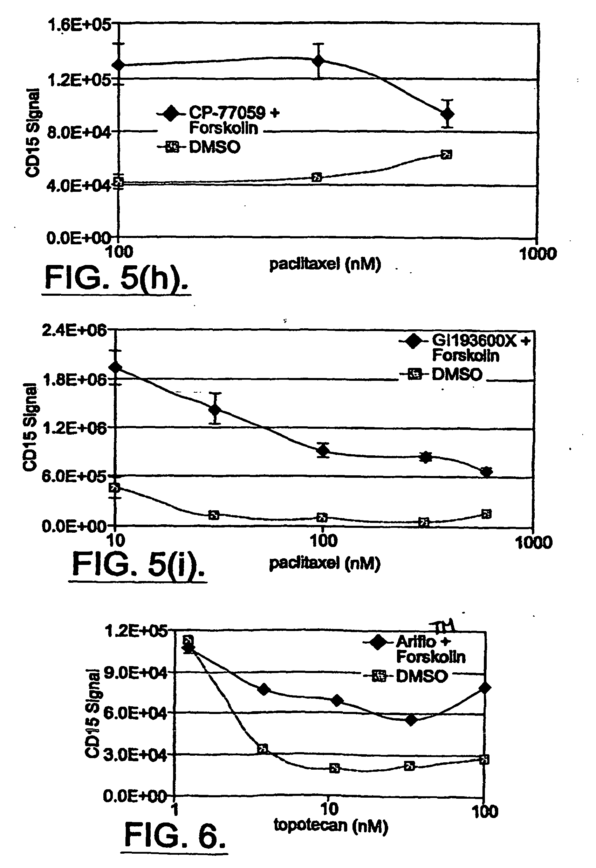

Neutrophil progenitors were protected by Ariflo™ plus forskotin from the cytotoxic effects of topotecan as measured by CELISA. Experimental details of this experiment are the same as described under Example 4 except that Ariflo™ (1 µM) was substituted for rolipram and topotecan was used as the anti-neoplastic agent. The results are depicted in Figure 6.

Example 6 illustrates the protection of neutrophil progenitors from the cytotoxic effects of topotecan by Ariflo™ plus forskolin as measured by the CEUSA method. Pretreatment of expanded CD34+ cells with Ariflo™ plus forskolin increased expression of CD15+ signal to a statistically significant level (p = 1.5 X 10⁻⁵), suggesting that more neutrophil progenitors survived the cytotoxic effects of the anti-neoplastic agent topotecan (Fig. 6).

### Example 7

### Protection of neutrophil progenitors as measured by CD 15 neutrophil antigen CELISA method -rolipram plus forskolin followed by treatment with anti-neoplastic agents listed in Table 1.

Protection of neutrophil progenitors from the cytotoxic effects of different anti-neoplastic agents, listed in table 1, by the PDE4 inhibitor, rolipram used in combination with forskolin was measured by CELISA method according to Example 4. The results are depicted in Table 1.

**TABLE 1**

| **Drug** | **Protection?** | **Target/mechanism** | **Cell cycle** |
|---|---|---|---|
| Paclitaxel | YES | microtubules, stabilizer | G2/M |
| Vinblastine | YES | tubulin, destabilizer | G2/M |
| Vinorelbine | YES | blocks polymerization of tubules | M |
| Etoposide | YES | stabilizes topoisomerase II enzyme | G2/M |
| 5-FU | YES | anti-metabolite, DNA syn inhibitor | S |
| methotrexate | YES | purine synthesis inhibitor | S, G2 |
| topotecan | YES | topoisomerase I inhibitor | G2/M, S |
| Cisplatin | NO | binds to DNA, X-link | non-specific |
| Carboplatin | NO | binds to DNA, different lesions than cisplatin : non-specific | |
| Doxorubicin | NO | intercalates DNA, stabilize topo II enzyme: non-specific | |

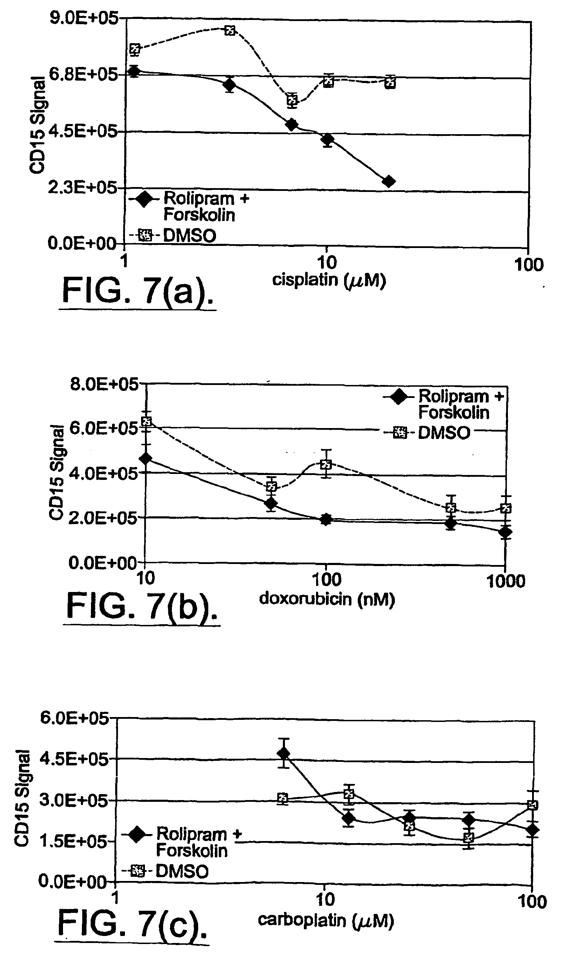

Protection of neutrophil progenitors from the cytotoxic effects of different anti-neoplastic agents by the PDE4 inhibitor rolipram in combination with forskolin was measured by CELISA method. The results are summarized in Table 1. Rolipram and forskolin protected the neutrophil progenitors from the agents that act in a cell cycle specific manner. In contrast, no protection was observed from the agents that bind DNA and act in a non-cell cycle specific manner. For instance, Figure 7 depicts results for non-cell specific neoplastic agents cisplatin, doxorubicin, and carboplatin pretreated with rolipram plus forskolin. For the effects of carboplatin the pretreatment of rolipram plus forskolin of the cells compared to DMSO showed no significant difference (p = 0.55). For cisplatin (p = 0.027) and doxorubicin (p = 0.0032), however the pretreatment with rolipram plus forskolin not only did not protect but appeared to significantly increase the toxicity towards the neutrophil progenitors.

Potential protection from mucositis induced by anti-neoplastic agents by pretreatment with PDE4 inhibitors is illustrated in Example 8. Following toxic insult by an anti-neoplastic agent, initial tissue damage and inflammation results in the death of proliferating epithelial cells. Tissue atrophy and ulceration are involved in the late phases of mucositis. An in vitro model using mink lung epithelial cells was used to examine whether a chemoprotection regimen including a PDE4 inhibitor would be useful for preventing mucositis. It is believed that a PDE4 inhibitor could potentially prevent mucositis in two ways: 1) by protecting epithelial progenitor cells and 2) by producing an anti-tumor necrosis factor effect (anti-inflammatory).

### Example 8

### Protection of mink lung epithelial cells by different PDE4 inhibitors in combination with forskolin from the cytotoxic effects of paclitaxel (10 nM).

CCL64 mink lung epithelial cells (ATCC # CCL-64) were grown in DMEM supplemented with 10% FBS and 1% penicillin-streptomycin and seeded into 96-well plates. After overnight incubation at 37°C, cells were treated with 100 µM forskolin in combination with each of the PDE4 inhibitors (1 µM). After 24 hours of treatment, paclitaxel was added to wells at various concentrations up to 10 nM. Time zero control plates were harvested by staining with Sulforhodamine B (Skehan P. et al., (1990), J. Nat'l Cancer Inst 82:1107-12.) After an additional 48 hours of incubation at 37°C, the plates were harvested and stained with sulforhodamine B. The absorbance of the samples was read at the optical density of 562 nm. The no-drug controls were set to 100% and values were normalized to time zero control. The results are depicted in Table II.

**TABLE II**

| PDE4 Inhibitor | % Increase in Survival |
|---|---|
| Rolipram | 110 |
| Ariflo™ | 110 |
| GI193600X | 120 |
| Picamilast | 130 |
| CDP-840¹ | 125 |
| Nitraquazone | 115 |
| CDP-840² | 110 |
| RS25344 | 105 |
| Denbufylline | 95 |
| CP-77059 | 85 |

| | |
|---|---|
| ¹ GR259653X | |
| ² GR259654X | |

As indicated in Example 8, cells were pre-treated with forskolin plus various PDE4 inhibitors (1 µM) and then challenged with paclitaxel at various concentrations.
The sulforhodamine B signal from the cells was expressed as percent of control (no-drug, vehide only). An increase in signal, indicating protection, ranged from 85% to 130% with the cells pre-treated with forskolin plus a particular PDE4 inhibitor and followed by a paclitaxel challenge at 10nM.

It is extremely important that an agent that protects normal cells against toxicity induced by anti-neoplastic agents should not impact the anti-tumor effects of the agent. In example 9, human cell lines representing colon, lung, and breast tumor types were tested with the chemoprotection regimen of either rolipram plus forskolin or Ariflo™ plus forskolin to examine whether they would or would not be protected from the anti-neoplastic effects of the cancer drug paclitaxel.

### Example 9

### No effect on the anti-tumor activity with human tumor cell lines HT-29 (colon), RKO (colon), lines A549 (lung), and MDA468 (breast) treated with forskolin plus rolipram or forskolin plus Ariflo™ followed by treatment with paclitaxel.

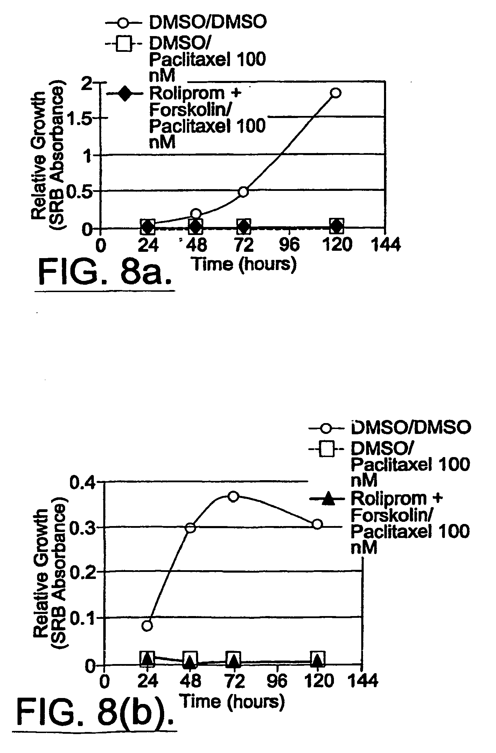

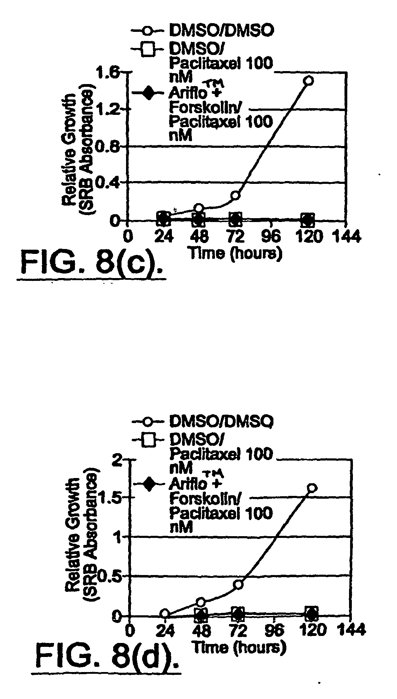

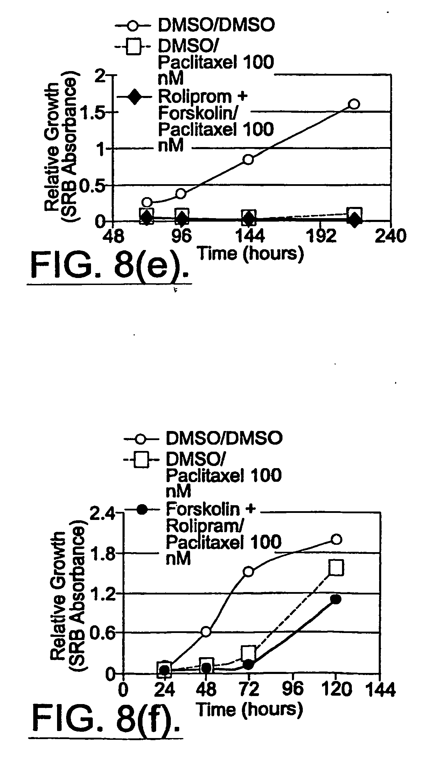

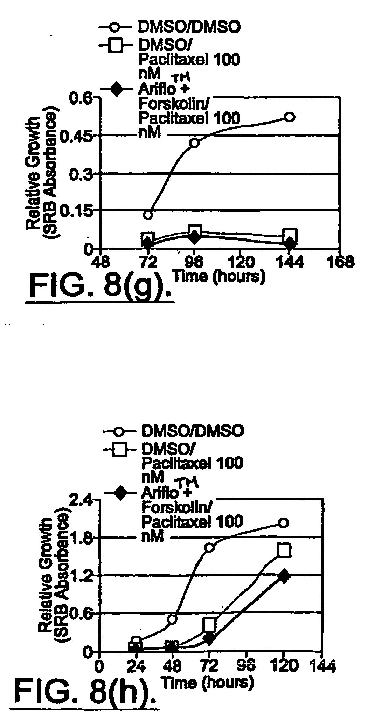

The human tumor cell lines HT-29 (colon: ATCC# HTB 38), RKO (colon, ATCC# HTB 130), lines A549 (lung, ATCC# CCL 185), and MDA468 (breast, ATCC# HTB 132) were obtained from the American Type Culture Collection and maintained in DMEM containing 10% FBS. Tumor cells were grown at 37°C in a humidified atmosphere of 5% CO₂. Adherent tumor cells were seeded into 96 well plates and incubated for six hours to allow for attachment. After six hours the chemoprotectants forskolin (100 µM) plus rolipram (1 µM) or forskolin (100 µM) plus Ariflo™ (1µM) were added. After overnight incubation, paclitaxel (100 nM) was added for 24 hours. Cells were then washed free of chemoprotectants and paclitaxel, placed in fresh media, and grown for 5 days. Growth was measured daily using sulforhodamine B (Skehan P. et. al., (1990), J. Nat'l Cancer Inst 82:1107-12). The results are depicted in Figure 8.

In Figure 8 (a), (b), (c) and (d), two different colon cancer lines (RKO, HT-29) were tested with either rolipram or Ariflo™ as the PDE4 inhibitor in the chemoprotection regimen (forskolin plus PDE4 inhibitor). The cells were then followed by a challenge by 100 nM paclitaxel. When challenged with paclitaxel there was no effect on the inhibition of tumour cell growth between cells receiving the chemoprotectant regimen versus those that were pre-treated with DMSO. The control tumor cells that were not exposed to paclitaxel quickly expanded over the 72 hours.

In Figure 8 (e), (f), (g), and (h) similar results were seen with a breast cancer cell line (MDA468, monitored for growth every other day for 10 days) and lung cancer cell line (A549). While the lung cell line A549 was not completely inhibited by paclitaxel, this cell line also had no additional protection with the chemoprotectant regimen of forskolin plus rolipram or forskolin plus Ariflo™. These data clearly show that the chemoprotectant regimen of PDE4 inhibitor and forskolin does not protect tumor cells from the anti-neoplastic effects of paclitaxel.

## Claims

1. A cancer treatment combination, comprising: therapeutically effective amounts of
(i) an anti-neoplastic agent that adversely affects normal progenitor cells upon use selected from paclitaxel, docetaxel, topotecan, irinotecan, 7-(4-methylpiperazino-methylene)-10,11-ethylenedioxy-20-camptothecin, vinblastine, vincristine, vinorelbine, etoposide, teniposide, fluorouracil, methotrexate, cytarabine, mercaptopurine, thioguanine, floxuridine, 5-fluorodeoxyuridine monophosphate, 5-azacytidine, gemcitabine, pentostatin, erythrohydroxynonyladenine, fludarabine phosphate and cladribine;
(ii) a PDE4 inhibitor selected from *cis*-4-cyano-4-[3-(cyclopentyloxy)-4-methoxyphenyl]cyclohexane-1-carboxylic acid or salt thereof, N-(3,5-dichloropyrid-4-yl)-3-cyclopentyloxy-4-methoxybenzamide, 1-[3-(cyclopentyloxy)-4-methoxyphenyl] ethanone (E)-O-{aminocarbonyl}oxime. (+/-4-[2-(3-cyclopentyloxy-4-methoxyphenyl)-2-phenylethyl]pyridine, nitroquazone, rolipram, denbufylline, 8-aza-1-(3-nitrophenyl)-3-(4-pyridylmethyl-2,4-quinazolinedione, 1-(3-carbomethoxyphenyl)-3-benzylpyrido [2,3d] pyrimidine-2,4(1H,3H)dione and methyl (3S,4S)-3-acetyl-4-[3-(cyclopentyloxy)-4-(methytoxy)phenyl]-3-methyl-1-pyrrolidinecarboxylate; and
(iii) an agent effective in raising intracellular concentrations of cAMP or analogs thereof selected from forskolin, dibutyryl cAMP, 8-bromo cAMP, terbutaline, albuterol, pirbuterol, bitolterol, metaproterenol, formoterol and salmeterol or salt thereof.

2. The combination of claim 1, wherein the PDE4 inhibitor is *cis*-4-cyano-4-[3-(cyclopentyloxy)-4-methoxyphenyl]cyclohexane-1-carboxylic acid or salt thereof.

3. The combination of daim 1, wherein the agent effective in raising intracellular concentrations of cAMP or analogs thereof is selected from forskolin and salmeterol or salt thereof.

4. The combination of claim 3, wherein the agent effective in raising intracellular concentrations of cAMP or analogs thereof is forskolin.

5. The combination of claim 1, wherein the anti-neoplastic agent that adversely affects normal progenitor cells upon use is selected from paclitaxel and topotecan.

6. Use of a combination according to any one of claims 1 to 5 in the preparation of a medicament for the treatment of cancer.

## Patentansprüche

1. Kombination zur Krebsbehandlung, umfassend: therapeutisch wirksame Mengen
(i) eines antineoplastischen Mittels, welches bei Verwendung nachteilig auf die normalen Progenitorzellen wirkt, ausgewählt aus Paclitaxel, Docetaxel, Topotecan, Irinotecan, 7-(4-Methylpiperazinomethylen)-10,11-ethylendioxy-20-camptothecin, Vinblastin, Vincristin, Vinorelbin, Etoposid, Teniposid, Fluoruracil, Methotrexat, Cytarabin, Mercaptopurin, Thioguanin, Floxuridin, 5-Fluordeoxyuridinmonophosphat, 5-Azacytidin, Gemcitabin, Pentostatin, Erythrohydroxynonyladenin, Fludarabinphosphat und Cladribin,
(ii) eines PDE4-Inhibitors, ausgewählt aus *cis*-4-Cyano-4-[3-(cyclopentyloxy)-4-methoxyphenyl]cyclohexan-1-carbonsäure oder eines Salzes davon, N-(3,5-Dichlorpyrid-4-yl)-3-cyclopentyloxy-4-methoxybenzamid, 1-[3-(Cyclopentyloxy)-4-methoxyphenyl]ethanon-(E)-O-{aminocarbonyl}oxim, (+/-)-4-[2-(3-Cyclopentyloxy-4-methoxyphenyl)-2-phenylethyl]pyridin, Nitroquazon, Rolipram, Denbufyllin, 8-Aza-1-(3-nitrophenyl)-3-(4-pyridylmethyl-2,4-chinazolindion, 1-(3-Carbomethoxyphenyl)-3-benzylpyrido[2,3-d]pyrimidin-2,4(1H,3H)dion und Methyl-(3S,4S)-3-acetyl-4-[3-(cyclopentyloxy)-4-(methyloxy)phenyl]-3-methyl-1-pyrrolidincarboxylat; und
(iii) eines Mittels, welches wirksam ist, intrazelluläre Konzentrationen von cAMP oder Analoga davon zu erhöhen, ausgewählt aus Forskolin, Dibutyryl-cAMP, 8-BromcAMP, Terbutalin, Albuterol, Pirbuterol, Bitolterol, Metaproterenol, Formoterol und Salmeterol oder einem Salz davon.

2. Kombination nach Anspruch 1, wobei der PDE4-Inhibitor *cis*-4-Cyano-4-[3-(cyclopentyloxy)-4-methoxyphenyl]cyclohexan-1-carbonsäure oder ein Salz davon ist.

3. Kombination nach Anspruch 1, wobei das Mittel, welches wirksam ist, intrazelluläre Konzentrationen von cAMP oder Analoga zu erhöhen, ausgewählt ist aus Forskolin und Salmeterol oder einem Salz davon.

4. Kombination nach Anspruch 3, wobei das Mittel, welches wirksam ist, intrazelluläre Konzentrationen von cAMP oder Analoga davon zu erhöhen, Forskolin ist.

5. Kombination nach Anspruch 1, wobei das antineoplastische Mittel, welches bei Verwendung nachteilig auf die normalen Progenitorzellen wirkt, ausgewählt ist aus Paclitaxel und Topotecan.

6. Verwendung einer Kombination gemäß einem der Ansprüche 1 bis 5 zur Herstellung eines Medikaments zur Behandlung von Krebs.

## Revendications

1. Combinaison pour le traitement du cancer, comprenant : des quantités thérapeutiquement efficaces
(i) d'un agent antinéoplasique qui a un effet néfaste sur les cellules souches normales lors de l'utilisation, choisi entre le paclitaxel, le docétaxel, le topotécan, l'irinotécan, la 7-(4-méthylpipérazino-méthylène)-10,11-éthylènedioxy-20-camptothécine, la vinblastine, la vincristine, la vinorelbine, l'étoposide, le téniposide, le fluoro-uracile, le méthotrexate, la cytarabine, la mercaptopurine, la thioguanine, la floxuridine, le monophosphate de 5-fluorodésoxy-uridine, la 5-azacytidine, la gemcitabine, la pentostatine, l'érythrohydroxynonyladénine, le phosphate du fludarabine et la cladribine ;
(ii) d'un inhibiteur de PDE4 choisi entre l'acide cis-4-cyano-4-[3-(cyclopentyloxy)-4-méthoxyphényl]cyclohexane-1-carboxylique ou un de ses sels, le N-(3,5-dichloropyride-4-yl)-3-cyclopentyloxy-4-méthoxybenzamide, la (E)-O-{aminocarbonyl}oxyme de 1-[3-(cyclopentyloxy)-4-méthoxyphényl]éthanone, la (+/-) -4-[2-(3-cyclopentyloxy-4-méthoxyphényl)-2-phényléthyl]pyridine, la nitroquazone, le rolipram, la denbufylline, la 8-aza-1-(3-nitrophényl)-3-(4-pyridylméthyl-2,4-quinazolinedione, la 1-(3-carbométhoxyphényl)-3-benzylpyrido[2,3d]pyrimidine-2,4(1H, 3H)dione et le (3S, 4S)-3-acétyl-4-[3-(cyclopentyloxy)-4-(méthyloxy)phényl]-3-méthyl-1-pyrrolidinecarboxylate de méthyle ; et
(iii) d'un agent efficace pour augmenter les concentrations intracellulaires de AMPc ou de ses analogues, choisi entre la forskoline, le dibutyryl-AMPc, le 8-bromo-AMPc, le terbutaline, l'albutérol, le pirbutérol, le bitoltérol, métaprotérénol, le formotérol et le salmétérol ou un de leurs sels.

2. Combinaison suivant la revendication 1, dans laquelle l'inhibiteur de PDE4 est l'acide *cis*-4-cyano-4-[3-(cyclopentyloxy)-4-méthoxyphényl]cyclohexane-1-carboxylique ou un de ses sels.

3. Combinaison suivant la revendication 1, dans laquelle l'agent efficace pour augmenter les concentrations intracellulaires d'AMPc ou de ses analogues est choisi entre la forskoline et le salmétérol ou un de leurs sels.

4. Combinaison suivant la revendication 3, dans laquelle l'agent efficace pour augmenter les concentrations intracellulaires d'AMPc ou de ses analogues est la forskoline.

5. Combinaison suivant la revendication 1, dans laquelle l'agent antinéoplasique qui a un effet néfaste sur les cellules souches normales lors de l'utilisation est choisi entre le paclitaxel et le topotécan.

6. Utilisation d'une combinaison suivant l'une quelconque des revendications 1 à 5 dans la préparation d'un médicament destiné au traitement du cancer.
